# EUROPEAN PATENT APPLICATION

(11) **EP 4 378 951 A1**
(43) Date of publication of application: **05.06.2024**
(21) Application number: 22848410.1
(22) Date of filing: 21.07.2022
(51) Int. Cl.: C07K 7/08, C12N 15/00, A61K 39/125, A61K 48/00

(54) **GENERAL AFFINITY EPITOPE POLYPEPTIDE FOR HUMAN RHINOVIRUS, AND ANTIBODY AND USES THEREOF**

(30) Priority: 26.07.2021 CN 202110843159
(71) Applicant: Beijing Wantai Biological Pharmacy Enterprise Co., Ltd., Beijing 102206 (CN); Xiamen University, Xiamen, Fujian 361005 (CN)
(72) Inventor: CHENG, Tong, Xiamen, Fujian 361005 (CN); ZHU, Rui, Xiamen, Fujian 361005 (CN); YANG, Hongwei, Xiamen, Fujian 361005 (CN); XU, Longfa, Xiamen, Fujian 361005 (CN); LIN, Yu, Xiamen, Fujian 361005 (CN); ZHOU, Zhenhong, Xiamen, Fujian 361005 (CN); YE, Xiangzhong, Beijing 102206 (CN); XIA, Ningshao, Xiamen, Fujian 361005 (CN)
(74) Representative: Vossius & Partner Patentanwälte Rechtsanwälte mbB
(86) International application number: PCT/CN2022/107144
(87) International publication number: WO 2023/005805

(57) **Abstract**

The present invention belongs to the field of immunobiology, and relates to an epitope polypeptide, such as a general affinity epitope polypeptide of human rhinovirus, and the use thereof. The present invention further relates to an antibody capable of binding to the epitope polypeptide and the use thereof. The present invention further relates to the use of the affinity epitope polypeptide or the antibody in the preparation of drugs or in methods for treating and/or preventing and/or diagnosing human rhinovirus and/or identifying a titer of human rhinovirus and/or identifying a titer of a neutralizing antibody of human rhinovirus. The affinity epitope polypeptide and antibody can be used in drugs or methods for treating and/or preventing and/or diagnosing human rhinovirus and/or identifying a titer of human rhinovirus and/or identifying a titer of a neutralizing antibody of human rhinovirus.

## Description

### Technical Field

The present invention relates to the field of immunology and molecular virology, especially the field of prevention and treatment for human rhinovirus. In particular, the present invention relates to a polypeptide useful for preventing or treating a human rhinovirus infection, a recombinant protein comprising such polypeptide and a carrier protein, and the use of such polypeptide and recombinant protein. The present invention also relates to an antibody against such polypeptide, and use thereof in the detection, prevention and/or treatment of a human rhinovirus infection and/or a disease caused by the infection.

### Background Art

Human Rhinovirus (hereinafter referred to as HRV) belongs to the genus *Enterovirus* in the family *Picornaviridae.* HRV is currently one of the viruses that infect humans and has the most serotypes. Based on the genetic characteristics of the virus, HRV can be divided into three major groups: A, B and C. According to the latest virus classification report (2019 version) released by the International Committee on Taxonomy of Viruses (ICTV), HRV-A includes 80 serotypes such as HRV-A1, 1B, 2, 7∼13; HRV-B includes 30 serotypes such as HRV-B3-6, 14, 17, 26~27; HRV-C includes a total of 55 serotypes including HRV-C1∼51, 54-57.

Patients with HRV infection are usually present with self-limiting common cold symptoms, with an average duration of about one week. However, in recent years, it has been found that HRV infection can also cause lower respiratory tract infections such as bronchiolitis and pneumonia, and is closely related to severe respiratory diseases such as acute wheezing, asthma exacerbation, persistent cough, dyspnea in children, and chronic obstructive pulmonary disease in adults. In addition, HRV can also cause co-infection with other respiratory viruses such as coronavirus, increasing patients' disease burden and risk of severe diseases (Li et al., 2018. J Infect. 76(3):311-13; David et al., 2020. JAMA. 323(20):2085-86). In addition to the mixed viral infections, HRV can also co-infect a host along with bacteria, leading to a series of diseases such as otitis media, sinusitis, and pharyngitis (Chistopher et al., 2017. Front Microbiol. 8: 2412). HRV can infect people of all ages, infants, young children, the elderly and people with low immunity are more susceptible to the infection, and the conditions are relatively serious. A certain level of immunity can be obtained after HRV infection, but the maintenance time is short, and there is almost no cross-protection between different serotypes of viruses.

HRV virus particles are non-enveloped, approximately round in shape, and have a diameter of approximately 27 to 30 nm, and its viral structure mainly comprises a peripheral capsid and an internal nucleic acid. The HRV virus capsid has a three-dimensional icosahedral symmetry and is composed of four structural proteins, VP1, VP2, VP3 and VP4, arranged according to certain rules. Among them, VP1 to VP3 are exposed on the surface of the viral capsid, and VP4 is buried on the inside of the viral capsid and is closely connected to the nucleic acid inside. The genome structures of different serotypes of HRV are similar. They are all single-stranded positive-sense RNA, with a full length of about 7200 bp. The entire viral genome of HRV contains only one open reading frame (ORF), and the 5' and 3' ends are untranslated regions (UTR) respectively. The unique ORF of HRV encodes a polyprotein of approximately 2200 amino acids in length, which is subsequently hydrolyzed by the virus-encoded protease into three precursor proteins, P1, P2, and P3. P1 can be further cleaved into 4 capsid proteins (VP4, VP2, VP1, VP3), P2 can be further cleaved into 3 non-structural proteins (2A, 2B, 2C), and P3 can be cleaved into 4 non-structural proteins (3A, 3B, 3C, 3D). 2C protein is one of the most conserved non-structural proteins among picornaviruses, which has ATPase activity and is involved in the rearrangement of host cell membrane proteins, the formation of viral replication complexes, as well as the evasion of host innate immunity (Oberste et al., 2004. J Gen Virol. 85: 1597; Du et al., 2015. Sci Rep. 5: 14302; Kevin et al., 2020. PNAS. 117(44):27598-607).

There are many types of HRV, and cross-immune reactivity between different serotypes is rare, which brings great difficulties to the prevention and treatment of HRV infection. It is of great significance to discover broad-spectrum affinity epitopes, obtain antibodies with broad-spectrum binding capabilities, and develop universal detection methods for HRV as well as effective preventive and therapeutic drugs (e.g., antibodies, vaccines, etc.) based thereon.

### Contents of the present invention

After extensive research, the inventors of the present application have identified a universal affinity epitope polypeptide in human rhinovirus, and the epitope polypeptide can induce an antibody having specific binding activity to at least 2 (e.g., 2, 5, 10, 15, 20, 24) subtypes of human rhinovirus. Based on this, the present application also provides a recombinant protein comprising the epitope polypeptide and a carrier protein, an antibody against the epitope polypeptide or recombinant protein, a cell strain expressing the antibody, as well as uses thereof.

### Isolated polypeptide or variant thereof

Therefore, in one aspect, the present application provides an isolated polypeptide or variant thereof, the polypeptide consists of at least 13 consecutive amino acid residues of human rhinovirus (HRV) 2C protein, and comprises the amino acid residue at positions 151 to 163 of the protein, and the variant differs from the polypeptide from which it is derived only by substitution of one or several (e.g., 1, 2, 3, 4, 5, 6, or 7) amino acid residues, and retains the biological function of the polypeptide from which it is derived (e.g., inducing an antibody having specific binding activity to at least 2 (e.g., 2, 5, 10, 15, 20, 24) subtypes of HRV).

In certain embodiments, the polypeptide consists of no more than 100 (e.g., no more than 95, no more than 90, no more than 85, no more than 80, no more than 75, no more than 70, no more than 65, no more than 60, no more than 55, no more than 50, no more than 45, no more than 40, no more than 35, or no more than 30) consecutive amino acid residues of the HRV 2C protein.

In certain embodiments, the polypeptide consists of 13 to 30 (e.g., 13 to 25, 13 to 21, 13 to 17, 13 to 15) consecutive amino acid residues of the HRV 2C protein.

In certain embodiments, the variant comprises a substitution (e.g., a conservative substitution) in an amino acid position corresponding to positions 156 and/or 159 of the HRV 2C protein.

Conservative substitutions are well known in the art and include, but are not limited to, changes such as: alanine to serine; arginine to lysine; asparagine to glutamine or histidine; aspartic acid to glutamic acid; cysteine to serine; glutamine to asparagine; glutamic acid to aspartic acid; glycine to proline; histidine to asparagine or glutamine; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine.

In certain embodiments, the polypeptide or variant thereof comprises a structure as represented by YSLPPX₁PKX₂FDGY (SEQ ID NO: 52);
wherein, X₁ is selected from: (i) amino acid residues D, A, S, N, and (ii) amino acid residues that are conservatively substituted relative to (i);
X₂ is selected from: (i) amino acid residues Y, H, and (ii) amino acid residues that are conservatively substituted relative to (i).

In certain embodiments, the HRV 2C protein has a sequence as set forth in SEQ ID NO: 9 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto.

In certain embodiments, the amino acid residues at positions 151 to 163 of the HRV 2C protein are as shown in any one of SEQ ID NOs: 1 and 30 to 34.

In certain embodiments, the polypeptide comprises or consists of amino acid residues at positions 151 to 163, amino acid residues at positions 149 to 165, amino acid residues at positions 147 to 167, or amino acid residues at positions 145 to 169 of the HRV 2C protein.

In certain embodiments, the polypeptide comprises or consists of a sequence as shown in any one of SEQ ID NOs: 1, 30 to 37.

In particular, the polypeptide or variant thereof of the present invention can be fused with a carrier protein to enhance the immunogenicity of the polypeptide or variant thereof, so that the polypeptide or variant thereof can be recognized by the immune system of an organism and induce an immune response in the organism.

Therefore, in another aspect, the present application provides a recombinant protein, which comprises the isolated polypeptide or variant thereof as described above, and a carrier protein, and the recombinant protein is not a naturally occurring protein or fragment thereof.

In certain embodiments, the polypeptide or variant thereof is linked to the carrier protein optionally via a linker (e.g., a rigid or flexible linker, such as a peptide linker comprising one or more glycines and/or one or more serines).

In certain embodiments, the carrier protein is selected from the group consisting of CRM197 protein or fragment thereof, HBcAg or fragment thereof, WHcAg or fragment thereof, keyhole hemocyanin, human serum albumin, bovine serum albumin, bovine thyroglobulin, ovalbumin.

In certain embodiments, the carrier protein is HBcAg or fragment thereof, and the amino acids at positions 79 to 80 of HBcAg are replaced with the polypeptide or variant thereof. In certain embodiments, the polypeptide or variant thereof is linked to HBcAg or fragment thereof via a linker. In certain embodiments, the fragment of HBcAg comprises or consists of aa 1 to 149 of HBcAg, for example, comprises a sequence as set forth in SEQ ID NO: 4. In certain embodiments, the recombinant protein has an amino acid sequence as set forth in any one of SEQ ID NO: 5, 44 to 51.

In another aspect, the present application provides an isolated nucleic acid molecule, which comprises a nucleotide sequence encoding the polypeptide or variant thereof or the recombinant protein as described above.

In another aspect, the present application provides a vector, which comprises the isolated nucleic acid molecule as described above.

In another aspect, the present application provides a host cell, which comprises the isolated nucleic acid molecule as described above or the vector as described above.

Such host cells include, but are not limited to, prokaryotic cells such as bacterial cells (e.g., *E. coli* cells), and eukaryotic cells such as yeast cells, insect cells, plant cells, and animal cells (e.g., mammalian cells, e.g., mouse cells, human cells, etc.).

In another aspect, the present application provides a method for preparing the polypeptide or variant thereof as described above or the recombinant protein as described above, which comprises culturing the host cell as described above under an appropriate condition, and recovering the polypeptide or variant thereof or the recombinant protein from a cell culture.

It can be understood that the polypeptide or variant thereof as described above or the recombinant protein as described above can be prepared by the above method, or can be obtained by artificial chemical synthesis, or other biochemistry or molecular biology methods known to those skilled in the art.

In another aspect, the present application provides a virus-like particle (VLP), wherein the isolated polypeptide or variant thereof as described above is displayed on its surface.

In certain embodiments, the virus-like particle comprises or consists of a fusion protein, and the fusion protein comprises the polypeptide or variant thereof as described above, and a carrier protein. In certain embodiments, the polypeptide or variant thereof is linked to the carrier protein via a linker.

In certain embodiments, the carrier protein is an HBcAg protein or fragment thereof (e.g., aa 1-149 of HBcAg). In certain embodiments, the amino acids at positions 79 to 80 of HBcAg are replaced with the polypeptide or variant thereof. In certain embodiments, the fusion protein has an amino acid sequence as set forth in any one of SEQ ID NO: 5, 44 to 51.

In another aspect, the present application provides a composition, which comprises the polypeptide or variant thereof, the recombinant protein, the isolated nucleic acid molecule, the vector, the host cell or the VLP as described above.

In certain embodiments, the composition is a pharmaceutical or immunogenic composition (e.g., a vaccine).

In certain embodiments, the composition further comprises a pharmaceutically acceptable carrier and/or excipient (e.g., adjuvant). In certain embodiments, the pharmaceutically acceptable carrier and/or excipient comprises an adjuvant. The adjuvant for co-administration or inclusion in the immunogenic composition according to the present invention should preferably be an adjuvant that is potentially safe, well tolerated and effective in humans. Such adjuvants are well known to those skilled in the art.

In certain embodiments, the composition comprises one or more kinds of the polypeptide or variant thereof, and these polypeptides or variants thereof may be independent or tandem, modified or unmodified, conjugated to other proteins or unconjugated to other proteins.

In certain embodiments, the composition comprises one or more kinds of the recombinant protein.

In certain embodiments, the composition comprises one or more kinds of the VLP.

The pharmaceutical composition or immunogenic composition as described above can be formulated into any dosage form known in the medical field, for example, tablets, pills, suspensions, emulsions, solutions, gels, capsules, powders, granules, elixirs, lozenges, suppositories, injections (including injection solutions, sterile powders for injection and concentrated solutions for injection), inhalants, sprays, etc. The preferred dosage form depends on the intended mode of administration and therapeutic use. The immunogenic composition of the present invention should be sterile and stable under the conditions of production and storage. One preferred dosage form is an injection. Such injection may be a sterile solution for injection. Additionally, the sterile solution for injection may be prepared as a sterile lyophilized powder (e.g., by vacuum drying or freeze drying) for ease of storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., solution containing 0.01% polysorbate 20), pH buffer solution (e.g., phosphate buffer solution), Ringer's solution and any combination thereof.

The pharmaceutical composition or immunogenic composition as described above may be administered by any suitable method known in the art, including, but not limited to, oral, buccal, sublingual, ophthalmic, topical, parenteral, rectal, intrathecal, intracytoplasmic reticulum, groin, intravesical, topically (e.g., powder, ointment, or drops), or nasal route. However, for many therapeutic uses, the preferred route/mode of administration is parenteral (e.g., intravenous or bolus injection, subcutaneous injection, intraperitoneal injection, intramuscular injection). Those skilled in the art should understand that the route and/or mode of administration will vary depending on the intended purpose.

The pharmaceutical composition or immunogenic composition as described above should be administered in an amount sufficient to induce an immune response against HRV. The appropriate amount of immunogen may be determined based on the specific disease or condition to be treated or prevented, its severity, the age of the subject, and other personal attributes of the particular subject (e.g., the general state of the subject's health and the robustness of the subject's immune system). Determination of effective doses is also guided by studies in animal models, followed by human clinical trials, and by administration regimens that significantly reduce the occurrence or severity of symptoms or conditions of the target disease in subjects.

In another aspect, the present application provides a use of the polypeptide or variant thereof, the recombinant protein, the isolated nucleic acid molecule, the vector, the host cell or the VLP as described above in the manufacture of a preparation for inducing an immune response against HRV in a subject and/ or for preventing and/or treating an HRV infection or a disease associated with HRV infection (e.g., respiratory tract infections, e.g., pneumonia, asthma) in a subject.

In certain embodiments, the preparation is a vaccine.

In another aspect, the present application provides a method for inducing an immune response against HRV in a subject and/or for preventing and/or treating an HRV infection or a disease associated with HRV infection (e.g., respiratory tract infection, such as pneumonia, asthma) in a subject, which comprises: administering an effective amount of the polypeptide or variant thereof, the recombinant protein, the VLP or the composition as described above to the subject in need thereof. In certain embodiments, the subject is a human.

For prophylactic applications, the polypeptide or variant thereof, the recombinant protein, the isolated nucleic acid molecule, the vector, the host cell or the VLP or the composition as described above is provided before any symptoms, for example before infection. Prophylactic administration is used to prevent or ameliorate any subsequent infection, to attenuate the expected severity, duration, or extent of infection and/or associated disease symptoms following exposure or suspected exposure to a virus or after actual onset of infection. Thus, in some embodiments, the subject to be treated is a subject who has or is at risk of developing an HRV infection, e.g., due to exposure or potential exposure to HRV.

For therapeutic applications, the polypeptide or variant thereof, the recombinant protein, the isolated nucleic acid molecule, the vector, the host cell or the VLP or the composition as described above is administered at or after the onset of symptoms of a disease or infection, for example after the onset of symptoms of an HRV infection, or after diagnosis of an HRV infection.

### Antibody and binding fragment thereof

In one aspect, the present application also provides a monoclonal antibody and antigen-binding fragment thereof, wherein the monoclonal antibody is capable of specifically binding to the polypeptide or variant thereof, the recombinant protein or the VLP as described above.

In certain embodiments, the monoclonal antibody is capable of specifically binding to the amino acid residues at positions 151 to 163 of the HRV 2C protein.

In certain embodiments, the monoclonal antibody or antigen-binding fragment thereof is selected from the group consisting of Fab, Fab', F(ab')₂, Fd, Fv, dAb, complementarity determining region fragment, single chain antibody (e.g., scFv), murine antibody, humanized antibody, fully human antibody, chimeric antibody (e.g., human-mouse chimeric antibody), or bispecific or multispecific antibody.

In certain embodiments, the antibody or antigen-binding fragment thereof comprises:
(a) a heavy chain variable region (VH) comprising the following three complementarity determining regions (CDRs):
   (i) a VH CDR1, which consists of the following sequence: SEQ ID NO: 27, or a sequence having a substitution, deletion or addition of one or several amino acids (e.g., a substitution, deletion or addition of 1, 2 or 3 amino acids) as compared thereto,
   (ii) a VH CDR2, which consists of the following sequence: SEQ ID NO: 28, or a sequence having a substitution, deletion or addition of one or several amino acids (e.g., a substitution, deletion or addition of 1, 2 or 3 amino acids) as compared thereto, and
   (iii) a VH CDR3, which consists of the following sequence: SEQ ID NO: 29, or a sequence having substitution, deletion or addition of one or several amino acids (e.g., substitution, deletion or addition of 1, 2 or 3 amino acids) as compared thereto;
      and/or,
(b) a light chain variable region (VL) comprising the following three complementarity determining regions (CDRs):
   (iv) a VL CDR1, which consists of the following sequence: SEQ ID NO: 24, or a sequence having a substitution, deletion or addition of one or several amino acids (e.g., a substitution, deletion or addition of 1, 2 or 3 amino acids) as compared thereto,
   (v) a VL CDR2, which consists of the following sequence: SEQ ID NO: 25, or a sequence having a substitution, deletion or addition of one or several amino acids (e.g., a substitution, deletion or addition of 1, 2 or 3 amino acids) as compared thereto, and
   (vi) a VL CDR3, which consists of the following sequence: SEQ ID NO: 26, or a sequence having a substitution, deletion or addition of one or several amino acids (e.g., a substitution, deletion or addition of 1, 2 or 3 amino acids) as compared thereto.

In certain embodiments, the CDRs described in any of (i) to (vi) are defined according to the IMGT numbering system.

In certain embodiments, the substitution as described in any one of (i) to (vi) is a conservative substitution.

In certain embodiments, the antibody or antigen-binding fragment thereof comprises:
(a) three heavy chain CDRs as follows: a VH CDR1 having a sequence as set forth in SEQ ID NO: 27, a VH CDR2 having a sequence as set forth in SEQ ID NO: 28, and a VH CDR3 having a sequence as set forth in SEQ ID NO: 29; and/or, three light chain CDRs as follows: a VL CDR1 having a sequence as set forth in SEQ ID NO: 24, a VL CDR2 having a sequence as set forth in SEQ ID NO: 25, and a VL CDR3 having a sequence as set forth in SEQ ID NO: 26;
   or,
(b) three CDRs as comprised in a heavy chain variable region (VH) as set forth in SEQ ID NO: 23; and/or, three CDRs as comprised in a light chain variable region (VL) as set forth in SEQ ID NO: 19.

In certain embodiments, the three CDRs comprised in the VH and/or the three CDRs comprised in the VL are defined by the Kabat, IMGT or Chothia numbering system.

In certain embodiments, the antibody or antigen-binding fragment thereof comprises:
(a) a heavy chain variable region (VH), which comprises an amino acid sequence selected from the following:
   (i) a sequence as set forth in SEQ ID NO: 23;
   (ii) a sequence having a substitution, deletion or addition of one or several amino acids (e.g., a substitution, deletion or addition of 1, 2, 3, 4 or 5 amino acids) as compared to the sequence as set forth in SEQ ID NO: 23; or
   (iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 23;
      and
(b) a light chain variable region (VL), which comprises an amino acid sequence selected from the following:
   (iv) a sequence as set forth in SEQ ID NO: 19;
   (v) a sequence having a substitution, deletion or addition of one or several amino acids (e.g., a substitution, deletion or addition of 1, 2, 3, 4 or 5 amino acids) as compared to the sequence as set forth in SEQ ID NO: 19; or
   (vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence as set forth in SEQ ID NO: 19.

In certain embodiments, the substitution as described in (ii) or (v) is a conservative substitution.

In certain embodiments, the antibody or antigen-binding fragment thereof comprises a VH comprising a sequence as set forth in SEQ ID NO: 23 and a VL comprising a sequence as set forth in SEQ ID NO: 19.

In certain embodiments, the antibody or antigen-binding fragment thereof is humanized.

In certain embodiments, the antibody or antigen-binding fragment thereof comprises a framework sequence derived from a human immunoglobulin. In certain embodiments, the antibody or antigen-binding fragment thereof comprises: a heavy chain framework sequence derived from a human heavy chain germline sequence, and a light chain framework sequence derived from a human light chain germline sequence.

In certain embodiments, the antibody or antigen-binding fragment thereof further comprises a constant region derived from a murine or human immunoglobulin. In certain embodiments, the heavy chain of the antibody or antigen-binding fragment thereof comprises a heavy chain constant region derived from a murine or human immunoglobulin (e.g., IgG1, IgG2, IgG3, or IgG4), and the light chain of the antibody or antigen-binding fragment thereof comprises a light chain constant region (e.g., a constant region of κ or λ chain) derived from a murine or human immunoglobulin.

On the other hand, the present application provides a monoclonal antibody and antigen-binding fragment thereof, which is produced by hybridoma cell strain 9A5 or a cell strain derived from hybridoma cell strain 9A5, wherein the hybridoma cell strain 9A5 is deposited in China Center for Type Culture Collection (CCTCC), and has the deposit number of CCTCC NO. C2021141.

In certain embodiments, the antibody or antigen-binding fragment thereof as described above specifically recognizes at least 2 (e.g., 2, 5, 10, 15, 20, 24) subtypes of HRV. In certain embodiments, the antibody or antigen-binding fragment thereof specifically recognizes one or more selected from the group consisting of HRV-A1B, HRV-A2, HRV-A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV-C15, and HRV-C23.

In certain embodiments, a representative strain of viral HRV-A1B is B632 (its GenBank accession number is D00239). In certain embodiments, a representative strain of viral HRV-A2 is HGP (its GenBank accession number is AB079139). In certain embodiments, a representative strain of viral HRV-A9 is 211 (its GenBank Accession number is AB079146). In certain embodiments, a representative strain of viral HRV-A16 is 11757 (its GenBank accession number is AB079152). In certain embodiments, a representative strain of virus HRV-A21 is 47 (its GenBank accession number is AB079157). In certain embodiments, a representative strain of viral HRV-A23 is 5124 (its GenBank accession number is AB079159). In certain embodiments, a representative strain of viral HRV-A29 is 5582 (its GenBank Accession number is AB079165). In certain embodiments, a representative strain of viral HRV-A34 is 137-3 (its GenBank accession number is AB079170). In certain embodiments, a representative strain of viral HRV-A36 is 324H (its GenBank accession number is AB079172). In certain embodiments, a representative strain of viral HRV-A40 is 1794 (its GenBank accession number is FJ445129). In certain embodiments, a representative strain of viral HRV-A43 is 58750 (its GenBank accession number is FJ445131). In certain embodiments, a representative strain of viral HRV-A49 is 8213 (its GenBank accession number is AB079185). In certain embodiments, a representative strain of viral HRV-A54 is F01-3774 (its GenBank accession number is AB079190). In certain embodiments, a representative strain of viral HRV-A59 is 611-CV35 (its GenBank accession number is AB079195). In certain embodiments, a representative strain of virus HRV-A77 is 130-63 (its GenBank accession number is FJ445154). In certain embodiments, a representative strain of viral HRV-A78 is 2030-65 (its GenBank accession number is FJ445183). In certain embodiments, a representative strain of virus HRV-A89 is 41467 (its GenBank accession number is M16248). In certain embodiments, a representative strain of viral HRV-B5 is Norman (its GenBank accession number is AB079142). In certain embodiments, a representative strain of viral HRV-B6 is Thompson (its GenBank accession number is AB079143). In certain embodiments, a representative strain of virus HRV-B14 is 1059 (its GenBank accession number is EU870450). In certain embodiments, a representative strain of virus HRV-B17 is 33342 (its GenBank accession number is EF173420). In certain embodiments, a representative strain of viral HRV-C11 is SC1065 (its GenBank accession number is KY369877). In certain embodiments, a representative strain of viral HRV-C15 is W10 (its GenBank accession number is GU219984). In certain embodiments, a representative strain of viral HRV-C23 is 8713-MY-10 (its GenBank accession number is KJ675506).

In another aspect, the present application provides an isolated nucleic acid molecule, which encodes the antibody or antigen-binding fragment thereof as described above, or a heavy chain variable region and/or light chain variable region thereof.

In another aspect, the present application provides a vector, which comprises the isolated nucleic acid molecule as described above. In certain embodiments, the vector is a cloning vector or an expression vector.

In another aspect, the present application provides a host cell, which comprises the isolated nucleic acid molecule as described above or the vector as described above.

On the other hand, the present application provides hybridoma cell strain 9A5, which is deposited in the China Center for Type Culture Collection (CCTCC) and has the deposit number of CCTCC NO. C2021141.

In another aspect, the present application provides a method for preparing the antibody or antigen-binding fragment thereof as described above, which comprises culturing the host cell or hybridoma cell as described above under a condition that allows expression of the antibody or antigen-binding fragment thereof, and recovering the antibody or antigen-binding fragment thereof from a cell culture of the host cell or hybridoma cell.

In another aspect, the present application provides a conjugate, which comprises the antibody or antigen-binding fragment thereof as described above, and a detectable label linked to the antibody or antigen-binding fragment thereof.

In certain embodiments, the detectable label is selected from the group consisting of enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide and biotin.

In another aspect, the present application provides a kit, which comprises the antibody or antigen-binding fragment thereof or the conjugate as described above.

In certain embodiments, the kit comprises the conjugate as described above.

In certain embodiments, the kit comprises the antibody or antigen-binding fragment thereof as described above, and a second antibody that specifically recognizes the antibody or antigen-binding fragment thereof. In certain embodiments, the second antibody is specific for an antibody from a species (e.g., murine or human) from which the constant region comprised in the antibody or antigen-binding fragment thereof of the present invention is derived. In certain embodiments, the second antibody is an anti-immunoglobulin (e.g., murine or human immunoglobulin) antibody, for example, an anti-IgG antibody. In certain embodiments, the second antibody is an anti-mouse IgG antibody or an anti-human IgG antibody. In certain embodiments, the second antibody further comprises a detectable label, such as an enzyme (e.g., horseradish peroxidase or alkaline phosphatase), a chemiluminescent reagent (e.g., acridinium esters, luminol and derivative thereof, or ruthenium derivative), a fluorescent dye (e.g., fluorescein or fluorescent protein), a radionuclide or a biotin.

In another aspect, the present application also provides a method for detecting the presence or level of HRV in a sample, which comprises using the antibody or antigen-binding fragment thereof as described above or the conjugate as described above.

In certain embodiments, the method is used for therapeutic purpose, diagnostic purpose, or non-therapeutic non-diagnostic purpose. For example, the method of the present invention can be applied to a subject to diagnose, prevent or treat a disease caused by a human rhinovirus in the subject. Furthermore, the method of the present invention may be applied to an in vitro sample (e.g., a cell culture, virus culture, etc.) to determine the presence or level of a rhinovirus in the sample, or the presence or potency of a rhinovirus neutralizing antibody.

In certain embodiments, the method is an immunological assay, such as a Western blot, an enzyme immunoassay (e.g., ELISA), a chemiluminescent immunoassay, a fluorescent immunoassay, or a radioimmunoassay.

In certain embodiments, the HRV comprises at least 2 (e.g., 2, 5, 10, 15, 20, 24) subtypes. In certain embodiments, the HRV comprises one or more selected from the group consisting of HRV-A1B, HRV-A2, HRV-A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV -C15, HRV-C23.

In certain embodiments, the method comprises using the conjugate as described above.

In certain embodiments, the method comprises using the antibody or antigen-binding fragment thereof as described above, and the method further comprises using a second antibody carrying a detectable label (e.g., enzyme (e.g., horseradish peroxidase or alkaline phosphate), chemiluminescent reagent (e.g., acridinium ester, luminol and derivative thereof, or ruthenium derivative), fluorescent dye (e.g., fluorescein or fluorescent protein), radionuclide, or biotin) to detect the antibody or antigen-binding fragment thereof.

In certain embodiments, the method comprises: (1) contacting the sample with the antibody or antigen-binding fragment thereof of the present invention; (2) detecting the formation of an antigen-antibody immune complex or detecting the amount of the immune complex. The formation of the immune complex indicates the presence of HRV or HRV-infected cell.

In another aspect, the present application provides a use of the antibody or antigen-binding fragment thereof as described above in the manufacture of a detection reagent, in which the detection reagent is used for detecting the presence or level of HRV in a sample, and/or for diagnosing whether a subject is infected with HRV.

In certain embodiments, the detection reagent detects the presence or level of HRV in a sample by the method as described above.

In certain embodiments, the sample is a body fluid sample (e.g., respiratory secretion, whole blood, plasma, serum, salivary excretion, or urine) from a subject (e.g., a mammal, preferably a human).

In another aspect, the present application provides a method for detecting the neutralizing activity of an HRV-neutralizing antibody or for screening for an HRV-neutralizing antibody, which comprises using the antibody or antigen-binding fragment thereof as described above or the conjugate as described above.

In certain embodiments, the method is an immunological assay, such as a Western blot, an enzyme immunoassay (e.g., ELISA), a chemiluminescent immunoassay, a fluorescent immunoassay, or a radioimmunoassay.

In certain embodiments, the method comprises: (i) incubating a test sample containing an antibody to be tested with HRV; (ii) incubating the product of step (i) with a cell; (iii) incubating the antibody or antigen-binding fragment thereof as described above or the conjugate as described above with the cell product of step (ii) (e.g., the cell product of step (ii) treated with membrane permeabilization); (iv) detecting the number of the cells labeled by the antibody or antigen-binding fragment thereof or the conjugate, and thereby determining the neutralizing activity of the antibody to be tested against HRV.

In another aspect, the present application provides a pharmaceutical composition, which comprises the antibody or antigen-binding fragment thereof as described above, and a pharmaceutically acceptable carrier and/or excipient.

In another aspect, the present application provides a use of the antibody or antigen-binding fragment thereof, the isolated nucleic acid molecule, the vector, the host cell or hybridoma cell as described above in the manufacture of a medicament for preventing and/or treating an HRV infection or a diseases related to HRV infection (e.g., respiratory tract infection, such as pneumonia, asthma) in a subject.

In certain embodiments, the subject is a mammal, such as a human.

In certain embodiments, the antibody or antigen-binding fragment thereof is used alone or in combination with an additional pharmaceutically active agent.

In another aspect, the present application provides a method for preventing and/or treating an HRV infection or a disease associated with HRV infection in a subject (e.g., a human), which comprises: administering an effective amount of the antibody or antigen-binding fragment thereof as described above or the pharmaceutical composition as described above to a subject in need thereof.

The antibody or antigen-binding fragment thereof or pharmaceutical composition of the present invention can be formulated into any dosage form known in the medical field, for example, tablets, pills, suspensions, emulsions, solutions, gels, capsules, powders, granules, elixirs, tablets, suppositories, injections (including solutions for injection, sterile powders for injection and concentrated solutions for injection), inhalants, sprays, etc. The preferred dosage form depends on the intended mode of administration and therapeutic use. The antibody or antigen-binding fragment thereof or pharmaceutical composition of the present invention should be sterile and stable under the conditions of production and storage. One preferred dosage form is an injection. Such injection may be a sterile solution for injection. For example, the sterile solution for injection may be prepared by incorporating a requisite dose of the antibody or antigen-binding fragment thereof of the present invention into in an appropriate solvent, and, optionally, incorporating other desired ingredients (including, but not limited to, pH adjuster, surfactant, adjuvant, ionic strength enhancer, isotonic agent, preservative, diluent, or any combination thereof) followed by sterilization by filtration. Additionally, the sterile solution for injection may be prepared as a sterile lyophilized powder (e.g., by vacuum drying or freeze drying) for ease of storage and use. Such sterile lyophilized powder can be dispersed in a suitable carrier before use, such as water for injection (WFI), bacteriostatic water for injection (BWFI), sodium chloride solution (e.g., 0.9% (w/v) NaCl), glucose solution (e.g., 5% glucose), surfactant-containing solution (e.g., solution containing 0.01% polysorbate 20), pH buffer solution (e.g., phosphate buffer solution), Ringer's solution and any combination thereof.

The antibody or antigen-binding fragments thereof of the present invention, or the pharmaceutical compositions of the present invention can be administered by any suitable method known in the art, including but not limited to, oral, buccal, sublingual, ophthalmic, topical, parenteral, rectal, intrathecal, intracytoplasmic reticulum, inguinal, intravesical, topical (e.g., powder, ointment, or drops), or nasal route. However, for many therapeutic uses, the preferred route/mode of administration is parenteral (e.g., intravenous or bolus injection, subcutaneous injection, intraperitoneal injection, intramuscular injection). The skilled artisan will understand that the route and/or mode of administration will vary depending on the intended purpose. In certain embodiments, the antibody or antigen-binding fragment thereof or pharmaceutical compositions of the present invention is administered by intravenous injection or bolus injection.

### Beneficial Effect

The present invention provides an epitope with broad-spectrum affinity for various HRV types, as well as an antibody against such epitope. The epitope peptide of the present invention can induce specific immune responses to at least 2 (e.g., 2, 5, 10, 15, 20, 24) subtypes of human rhinovirus; the antibody of the present invention exhibits broad-spectrum binding capability to various types of HRV, and can be used to detect, prevent and/or treat a human rhinovirus infection and/or a disease caused by such infection (e.g., respiratory infection).

### Definition of Terms

In the present invention, unless otherwise stated, the scientific and technical terms used herein have the meanings commonly understood by those skilled in the art. Moreover, the virology, biochemistry, and immunology laboratory procedures used in this article are routine procedures widely used in the corresponding fields. Meanwhile, in order to better understand the present invention, definitions and explanations of relevant terms are provided below.

As used herein, when referring to the amino acid position of HRV non-structural protein 2C, the description is made by referring to the amino acid sequence of non-structural protein 2C of HRV-C15 strain (W10) (the sequence as set forth in SEQ ID NO: 9). For example, the expression "amino acid residues at positions 151 to 163 of HRV non-structural protein 2C (aa 151-163)" refers to the amino acid residues at positions 151 to 163 of the polypeptide as set forth in SEQ ID NO: 9. However, those skilled in the art understand that in the amino acid sequence of HRV non-structural protein 2C, mutation or variation (including but not limited to, substitution, deletion and/or addition, such as different types of HRV) may occur naturally or be introduced artificially, and does not affect its biological function. For example, among different strains of rhinovirus (e.g., HRV-A1B, HRV-A2, HRV-A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV-C15 or HRV-C23), the amino acid sequence of non-structural protein 2C may have naturally occurring difference (mutation or variation) without affecting its biological function. Therefore, in the present invention, the term "HRV non-structural protein 2C" shall include all such sequences, including, for example, the sequence as set forth in SEQ ID NO: 9 and its natural or artificial variants. And, when describing the sequence fragment of rhinovirus non-structural protein 2C, it includes not only the sequence fragment of SEQ ID NO: 9, but also the corresponding sequence fragment in its natural or artificial variants. For example, the expression "aa 151-163 of rhinovirus non-structural protein 2C" includes amino acid residues at positions 151 to 163 of SEQ ID NO: 9, and corresponding fragments in its (natural or artificial) variants. According to the present invention, the expression "corresponding sequence fragment" or "corresponding fragment" refers to a fragment located at equivalent positions in the sequence being compared, when the sequence is optimally aligned, that is, when the sequence is aligned to obtain the highest percent identity.

In addition, according to the complete genome of HRV-C15 strain (GU219984), it is known that aa 151-163 of HRV non-structural protein 2C (e.g., the sequence as set forth in SEQ ID NO: 1) corresponds to aa 1238-1250 of the polyprotein translated from the rhinovirus genome. Therefore, in the present invention, the expression "aa 151-163 of HRV non-structural protein 2C" and the expression "aa 1238-1250 of the polyprotein translated from the HRV genome" have equivalent meanings and can be used interchangeably.

As used herein, the term "HBcAg" refers to a core antigenic protein of hepatitis B virus (HBV), which is well known to those skilled in the art (see, for example, NCBI GENBANK database accession number: AAO63517.1). When referring to the amino acid position of HBcAg, reference is made to the sequence as set forth in SEQ ID NO: 4. For example, the expression "amino acid residues at positions 1 to 149 of HBcAg (aa 1-149)" refers to the amino acid residues at positions 1 to 149 of the polypeptide as set forth in SEQ ID NO: 4. However, those skilled in the art understand that in the amino acid sequence of HBcAg, mutation or variation (including, but not limited to, substitution, deletion and/or addition, such as different gene types or gene subtypes of HBcAg) may occur naturally or be introduced artificially, without affecting its biological function. Therefore, in the present invention, the term "HBcAg" shall include all such sequences, including, for example, the sequence as set forth in SEQ ID NO: 4 and natural or artificial variants thereof. And, when describing a sequence fragment of HBcAg, it includes not only the sequence fragment of SEQ ID NO: 4, but also the corresponding sequence fragment of its natural or artificial variants. For example, the expression "amino acid residues at positions 1 to 149 of HBcAg" includes the amino acid residues at positions 1 to 149 of SEQ ID NO: 4, and corresponding fragments in its (natural or artificial) variants. According to the present invention, the expression "corresponding sequence fragment" or "corresponding fragment" refers to a fragment located at equivalent positions in the sequence being compared, when the sequence is optimally aligned, that is, when the sequence is aligned to obtain the highest percent identity.

As used herein, the term "WHcAg" refers to a core protein of woodchuck hepatitis virus, which is well known to those skilled in the art (see, for example, NCBI GENBANK database accession number: ADE19018.1).

As used herein, the term "CRM197 (Cross-Reacting Materials 197)" refers to a nontoxic mutant of diphtheria toxin (DT) that differs from wild-type diphtheria toxin in that the amino acid residue at position 52 is substituted from Gly to Glu (G. Giannini, R. Rappuoli, G. Ratti et al., Nucleic Acids Research. 1984. 12: 4063-4070). Diphtheria toxin is well known to those skilled in the art (see, for example, Choe S, Bennett M, Fujii G, et al., Nature. 1992. 357:216-222), and its amino acid sequence can be found, for example, in GenBank accession number AAV70486.1.

As used herein, the term "identity" is used to refer to the match of sequences between two polypeptides or between two nucleic acids. To determine the percent identity of two amino acid sequences or two nucleic acid sequences, the sequences are aligned for optimal comparison purposes (e.g., gaps may be introduced in the first amino acid sequence or nucleic acid sequence to best match the second amino acid or nucleic acid sequence). The amino acid residues or nucleotides at the corresponding amino acid positions or nucleotide positions are then compared. When a position in the first sequence is occupied by the same amino acid residue or nucleotide as the corresponding position in the second sequence, the molecules are identical at the position. The percent identity between two sequences is a function of the number of identical positions shared by the sequences (i.e., percent identity = number of identical overlapping positions/total number of positions × 100%). In certain embodiments, both sequences have the same length.

The determination of percent identity between two sequences can also be accomplished using mathematical algorithms. One non-limiting example of mathematical algorithm for comparison of two sequences is the algorithm of Karlin and Altschul, 1990, Proc. Natl. Acad. Sci. U.S.A. 87:2264-2268, as well as the improvement in Karlin and Altschul, 1993, Proc. Natl. Acad. Sci. U.S.A. 90:5873-5877. Such algorithms are integrated into the NBLAST and XBLAST programs of Altschul et al., 1990, J. Mol. Biol. 215:403.

As used herein, the term "variant", in the context of polypeptides (including polypeptides), also refers to a polypeptide or peptide comprising an amino acid sequence that has been altered by introducing substitution, deletion, or addition of amino acid residues. As used herein, the term "variant" also refers to a polypeptide or peptide that has been modified (i.e., by covalently linking any type of molecule to the polypeptide or peptide). For example, and without limitation, polypeptides may be modified, for example, by glycosylation, acetylation, PEGylation, phosphorylation, amidation, derivatization with known protecting/blocking groups, proteolytic cleavage, attachment to cellular ligands or other proteins, etc. Derivatized polypeptides or peptides can be produced by chemical modification using techniques known to those skilled in the art, including, but not limited to, specific chemical cleavage, acetylation, formylation, metabolic synthesis of tunicamycin, and the like. Furthermore, a variant has a similar, identical or improved function to the polypeptide or peptide from which it is derived.

As used herein, the term "fragment" refers to a peptide or polypeptide that contains at least 3 consecutive amino acid residues, at least 4 consecutive amino acid residues, at least 5 consecutive amino acid residues, at least 6 consecutive amino acid residues, at least 7 consecutive amino acid residues, at least 8 consecutive amino acid residues, at least 9 consecutive amino acid residues, at least 10 consecutive amino acid residues, at least 11 consecutive amino acid residues, at least 12 consecutive amino acid residues, at least 13 consecutive amino acid residues, at least 14 consecutive amino acid residues, at least 15 consecutive amino acid residues, at least 16 consecutive amino acid residues, at least 20 consecutive amino acid residues, at least 25 consecutive amino acid residues, at least 30 consecutive amino acid residues, or more amino acid residues of the amino acid sequence of another polypeptide. In specific embodiments, the fragment of a polypeptide retains at least one function of the polypeptide.

As used herein, the term "epitope" refers to a portion of an antigen capable of being recognized and specifically bound by a particular antibody. When the antigen is a polypeptide, the epitope can be formed from consecutive amino acids or non-consecutive amino acids neighbored by the tertiary folding of the protein, called linear epitope or conformational epitope, respectively. The epitope formed from consecutive amino acids is typically retained upon protein denaturation, whereas the epitope formed from tertiary folding is typically lost upon protein denaturation. The epitope usually comprises at least 3, 4, 5, 6, 7, 8, 9, 10, 11, 12, 13, 14 or 15 consecutive or non-consecutive amino acids in a unique spatial conformation.

As used herein, the term "epitope peptide" refers to a peptide segment capable of functioning as an epitope on an antigen. In some cases, an epitope peptide alone is capable of being specifically recognized/bound by an antibody directed against that epitope. In other cases, it may be necessary to fuse the epitope peptide to a carrier protein so that the epitope peptide can be recognized by a specific antibody. As used herein, the term "carrier protein" refers to a protein that can act as a carrier for an epitope peptide, i.e., it can be inserted by the epitope peptide at a specific position (e.g., within the protein, at N-terminus or at C-terminus), so that the epitope peptide can be presented and recognized by the antibody or immune system. Such carrier proteins are well known to those skilled in the art and include, for example, HPV L1 protein (the epitope peptide can be inserted between amino acids at positions 130 to 131 or between amino acids at positions 426 to 427 of the protein, see, Slupetzky, K. et al., J Gen Virol, 2001, 82: 2799-2804; Varsani, A. et al., J Virol, 2003, 77: 8386-8393), HBV core antigen (the amino acids at positions 79 to 81 of the protein can be replaced by the epitope peptide, see, Koletzki, D., et al. Biol Chem, 1999, 380: 325-333), woodchuck hepatitis virus core protein (the amino acids at positions 79 to 81 of the protein can be replaced by the epitope peptide, see, Sabine König, J. Virol. 1998, 72(6):4997), CRM197 protein (the epitope peptide can be connected to the N-terminus or C-terminus of the protein or its fragment). Optionally, a linker can be used between the epitope peptide and the carrier protein to facilitate folding of each.

As used herein, the term "antibody" refers to an immunoglobulin molecule typically composed of two pairs of polypeptide chains, each pair having a light chain (LC) and a heavy chain (HC). Antibody light chains can be classified into κ (kappa) and λ(lambda) light chains. Heavy chains can be classified as mu, delta, gamma, alpha, or epsilon, and define the antibody's isotype as IgM, IgD, IgG, IgA, and IgE, respectively. Within the light and heavy chains, the variable and constant regions are connected by a "J" region of approximately 12 or more amino acids, and the heavy chain also contains a "D" region of approximately 3 or more amino acids. Each heavy chain consists of a heavy chain variable region (VH) and a heavy chain constant region (CH). The heavy chain constant region consists of 3 domains (CH1, CH2 and CH3). Each light chain consists of a light chain variable region (VL) and a light chain constant region (CL). The light chain constant region consists of one domain, CL. Constant domains are not directly involved in the binding of antibodies to antigens, but exhibit a variety of effector functions, such as mediating the binding of immunoglobulins with host tissues or factors, including various cells of the immune system (e.g., effector cells) and the first component of the classical complement system (C1q). The VH and VL regions can also be subdivided into highly variable regions called complementarity-determining regions (CDRs), interspersed with more conservative regions called framework regions (FRs). Each VH and VL consists of 3 CDRs and 4 FRs arranged from the amino terminus to the carboxyl terminus in the following order: FR1, CDR1, FR2, CDR2, FR3, CDR3, FR4. The variable regions (VH and VL) of each heavy chain/light chain pair respectively form the antigen-binding site. The assignment of amino acids to each region or domain may follow the definition as described in Kabat, Sequences of Proteins of Immunological Interest (National Institutes of Health, Bethesda, Md. (1987 and 1991)), or Chothia & Lesk (1987) J. Mol. Biol. 196 :901-917; Chothia et al. (1989) Nature 342:878-883.

As used herein, the term "complementarity determining region" or "CDR" refers to the amino acid residues in the variable region of an antibody that are responsible for antigen binding. The variable regions of the heavy chain and light chain each contain three CDRs, named CDR1, CDR2 and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883) or the IMGT numbering system (Lefranc et al. al., Dev. Comparat. Immunol. 27:55-77, 2003). For a given antibody, one skilled in the art will readily identify the CDRs defined by each numbering system. Moreover, the correspondence between different numbering systems is well known to those skilled in the art (e.g., see Lefranc et al., Dev. Comparat. Immunol. 27:55-77, 2003).

In the present invention, the CDRs contained in the antibody or antigen-binding fragment thereof of the present invention can be determined according to various numbering systems known in the art. In certain embodiments, the CDRs contained in the antibody or antigen-binding fragment thereof of the present invention are preferably determined by the Kabat, Chothia, or IMGT numbering systems. In certain embodiments, the CDRs contained in the antibody or antigen-binding fragment thereof of the present invention are preferably determined by the IMGT numbering system.

As used herein, the term "framework region" or "FR" residues refers to those amino acid residues in antibody variable region other than the CDR residues as defined above.

The term "antibody" is not limited to any particular method of producing the antibody, but includes, for example, recombinant antibody, monoclonal antibody, and polyclonal antibody. The antibody may be of different isotypes, for example, IgG (e.g., IgG1, IgG2, IgG3 or IgG4 subtypes), IgA1, IgA2, IgD, IgE or IgM antibody.

As used herein, the term "antigen-binding fragment" of antibody refers to a polypeptide comprising a fragment of a full-length antibody that retains the ability to specifically bind to the same antigen to which the full-length antibody binds, and/or competes with the full-length antibody for specific binding to the antigen, which is also called "antigen-binding moiety." See generally, Fundamental Immunology, Ch. 7 (Paul, W., ed., 2nd ed., Raven Press, N.Y. (1989), which is incorporated herein by reference in its entirety for all purposes. Antigen-binding fragments of antibody can be obtained by recombinant DNA technology or by enzymatic or chemical cleavage of intact antibody. Non-limiting examples of antigen-binding fragment include Fab, Fab', F(ab')₂, Fd, Fv, complementarity determining region (CDR) fragment, scFv, diabody, single domain antibody, chimeric antibody, linear antibody, nanobody (technology from Domantis), probody and such polypeptide, which contains at least a portion of antibody sufficient to confer specific antigen-binding capability to the polypeptide. Engineered antibody variants are reviewed in Holliger et al., 2005; Nat Biotechnol, 23: 1126-1136.

As used herein, the term "full-length antibody" refers to an antibody consisting of two "full-length heavy chains" and two "full-length light chains." Wherein, "full-length heavy chain" refers to a polypeptide chain that consists of a heavy chain variable region (VH), a heavy chain constant region CH1 domain, a hinge region (HR), a heavy chain constant region CH2 domain and a heavy chain constant region CH3 domain in the direction from the N-terminal to the C-terminal; and, when the full-length antibody is of IgE isotype, it optionally further comprises a heavy chain constant region CH4 domain. Preferably, a "full-length heavy chain" is a polypeptide chain consisting of VH, CH1, HR, CH2 and CH3 in the direction from N-terminal to C-terminal. A "full-length light chain" is a polypeptide chain consisting of a light chain variable region (VL) and a light chain constant region (CL) in the direction from N-terminal to C-terminal. The two pairs of full-length antibody chains are linked together by disulfide bonds between CL and CH1 and disulfide bonds between the HRs of two full-length heavy chains. The full-length antibody of the present invention can be derived from a single species, such as human; it can also be a chimeric antibody or a humanized antibody. The full-length antibody of the present invention contains two antigen-binding sites formed by VH and VL pairs respectively, and these two antigen-binding sites specifically recognize/bind the same antigen.

As used herein, the term "Fd" refers to an antibody fragment consisting of VH and CH1 domains; the term "dAb fragment" refers to an antibody fragment consisting of VH domain (Ward et al., Nature 341:544 546 (1989)); the term "Fab fragment" refers to an antibody fragment consisting of VL, VH, CL and CH1 domains; the term "F(ab')₂ fragment" refers to an antibody fragment consisting of two Fab fragments connected by a disulfide bridge on the hinge region; the term "Fab' fragment" refers to a fragment obtained by reducing the disulfide bond connecting two heavy chain fragments in F(ab')₂ fragment, consisting of a complete light chain and a Fd of heavy chain (consisting of VH and CH1 domains).

As used herein, the term "Fv" refers to an antibody fragment consisting of VL and VH domains of a single arm of antibody. Fv fragment is generally considered to be the smallest antibody fragment that can form a complete antigen-binding site. It is generally believed that six CDRs confer the antigen-binding specificity on an antibody. However, even a variable region (e.g., a Fd fragment, which comprises only three antigen-specific CDRs) can also recognize and bind to antigen, although its affinity may be lower than that of complete binding site.

As used herein, the term "Fc" refers to an antibody fragment formed with the second and third constant regions of the first heavy chain and the second and third constant regions of the second heavy chain via disulfide bonding in an antibody. The Fc fragment of antibody has many different functions but does not participate in antigen binding.

As used herein, the term "scFv" refers to a single polypeptide chain comprising VL and VH domains, in which the VL and VH are connected by a linker (see, for example, Bird et al., Science 242:423 -426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecule may have the general structure: NH₂-VL-linker-VH-COOH or NH₂-VH-linker-VL-COOH. Suitable linker in the prior art consists of repeated GGGGS amino acid sequences or variants thereof. For example, a linker having the amino acid sequence (GGGGS)₄ can be used, but variants thereof can also be used (Holliger et al. (1993), Proc. Natl. Acad. Sci. USA 90: 6444-6448). Other linkers useful in the present invention are described by Alfthan et al. (1995), Protein Eng. 8:725-731; Choi et al. (2001), Eur. J. Immunol. 31:94-106; Hu et al. (1996), Cancer Res. 56:3055-3061; Kipriyanov et al. (1999), J. Mol. Biol. 293:41-56; and Roovers et al. (2001), Cancer Immunol. In some cases, a disulfide bond may also exist between VH and VL of scFv. In certain embodiments of the present invention, scFv can form di-scFv, which refers to an antibody formed with two or more individual scFvs connected in series. In certain embodiments of the present invention, scFv can form (scFv)₂, which refers to an antibody formed with two or more individual scFvs connected in parallel.

Each of the above antibody fragments retains the ability to specifically bind to the same antigen to which the full-length antibody binds, and/or competes with the full-length antibody for specific binding to the antigen.

Antigen-binding fragments of antibody (e.g., the above-described antibody fragments) can be obtained from a given antibody (e.g., the antibody provided by the present invention) using conventional techniques known to those skilled in the art (e.g., recombinant DNA technology or enzymatic or chemical fragmentation methods), and the antigen-binding fragments of antibody are screened for specificity in the same manner as for intact antibodies.

As used herein, when the term "antibody" is mentioned, it includes not only intact antibody but also antigen-binding fragments of the antibody, unless the context clearly indicates otherwise.

As used herein, the term "chimeric antibody" refers to an antibody in which part of the light chain and/or heavy chain is derived from an antibody (which may be derived from a specific species or belong to a specific antibody class or subclass), and the other part of the light chain or/and heavy chain is derived from another antibody (which may be derived from the same or different species or belong to the same or different antibody class or subclass), nevertheless, it still retains binding activity to the target antigen (U.S.P 4,816,567 to Cabilly et al.; Morrison et al., Proc. Natl. Acad. Sci. USA, 81:6851 6855 (1984)). In certain embodiments, the term "chimeric antibody" may include an antibody (e.g., a human-mouse chimeric antibody) in which the heavy and light chain variable regions of the antibody are derived from a first antibody (e.g., a murine antibody), and the heavy and light chain constant regions of the antibody are derived from a secondary antibody (e.g., a human antibody).

As used herein, the term "humanized antibody" refers to a non-human antibody that has been genetically engineered and whose amino acid sequence has been modified to increase sequence homology to a human antibody. Generally speaking, all or part of the CDR regions of a humanized antibody come from a non-human antibody (donor antibody), and all or part of the non-CDR regions (e.g., variable region FR and/or constant region) come from a human immunoglobulin (receptor antibody). Typically, at least one or two, but usually all three receptor CDRs (of the heavy and/or light immunoglobulin chains) of a humanized antibody are replaced by donor CDRs. The immunoglobulin that provides CDRs is called "donor" and the immunoglobulin that provides framework is called "receptor." In one embodiment, the donor immunoglobulin is a non-human (e.g., rabbit) antibody, and the receptor framework can be a naturally occurring human framework, or a sequence having an identity of about 85%, 90%, 95%, 99%, or higher as compared thereto. A humanized antibody generally retains the expected properties of donor antibody, including but not limited to, antigen specificity, affinity, reactivity, etc. The donor antibody may be a mouse, rat, rabbit, or non-human primate antibody with desired properties (e.g., antigen specificity, affinity, reactivity, etc.).

The chimeric antibody or humanized antibody of the present invention can be prepared based on a sequence of a monoclonal antibody produced by immunizing an animal (e.g., a mouse). The DNAs encoding heavy and light chains can be obtained from target hybridomas or specific B cells from the immunized animals and engineered to contain human immunoglobulin sequences using standard molecular biology techniques.

To prepare a chimeric antibody, immunoglobulin variable regions from an immunized animal (e.g., a mouse) can be linked to human immunoglobulin constant regions using methods known in the art (see, for example, Cabilly et al., U.S. Patent No. 4,816,567). For example, DNA encoding VH is operably linked to another DNA molecule encoding a heavy chain constant region to obtain a full-length heavy chain gene. The sequences of human heavy chain constant region genes are known in the art (see, for example, Kabat, E.A. et al. (1991), Sequences of Proteins of Immunological Interest, Fifth Edition, U.S. Department of Health and Human Services, NIH Publication No. 91-3242), and DNA fragments containing these regions can be obtained by standard PCR amplification. The heavy chain constant region may be an IgG1, IgG2, IgG3, IgG4, IgA, IgE, IgM or IgD heavy chain constant region, but is generally preferred to be an IgG1 or IgG4 constant region. For example, DNA encoding VL is operably linked to another DNA molecule encoding a light chain constant region CL to obtain a full-length light chain gene (as well as a Fab light chain gene). The sequences of human light chain constant region genes are known in the art (see, for example, Kabat, E.A. et al. (1991), Sequences of Proteins of Immunological Interest, Fifth Edition, U.S. Department of Health and Human Services, NIH Publication No. 91-3242), and DNA fragments containing these regions can be obtained by standard PCR amplification. The light chain constant region may be a constant region of κ or λ light chain, but a constant region of κ light chain is generally preferred.

To prepare humanized antibodies, CDR regions from an immunized animal (e.g., a mouse) can be grafted into human framework sequences using methods known in the art (see, Winter, U.S. Patent No. 5,225,539; Queen et al., U.S. Patent Nos. 5,530,101; 5,585,089; 5,693,762 and 6,180,370; and Lo, Benny, K.C., editor, in Antibody Engineering: Methods and Protocols, volume 248, Humana Press, New Jersey, 2004). The human framework sequences may be derived from, for example, a germline antibody gene. The term "germline antibody gene" refers to a sequence encoding an immunoglobulin present in the genome of an organism that has not undergone a maturation process comprising a genetic rearrangement or mutation that would lead to the expression of specific immunoglobulin. "Heavy chain germline gene" refers to a germline antibody gene or gene fragment encoding an immunoglobulin heavy chain, which comprises V (variable) gene, D (diversity) gene, J (joining) gene and C (constant) gene; similarly, the expression "light chain germline gene" refers to a germline antibody gene or gene fragment encoding an immunoglobulin light chain, which comprises V (variable) gene, J (joining) gene and C (constant) gene. In the present invention, the amino acid sequence encoded by the germline antibody gene or germline antibody gene fragment is also called "germline sequence", and the amino acid sequence encoded by the heavy chain germline gene is called heavy chain germline sequence, and the amino acid sequence encoded by the light chain germline gene is called the light chain germline sequence. Germline antibody genes or germline antibody gene fragments and their corresponding germline sequences are well known to those skilled in the art and can be obtained or queried from professional databases (e.g., IMGT, UNSWIg, NCBI or VBASE2).

As used herein, the term "specific binding" refers to a non-random binding reaction between two molecules, such as the reaction between an antibody and the antigen it targets. The strength or affinity of a specific binding interaction can be expressed in terms of the equilibrium dissociation constant (K_{D}) of the interaction. In the present invention, the term "K_{D}" refers to the dissociation equilibrium constant of a specific antibody-antigen interaction, which is used to describe the binding affinity between an antibody and an antigen. The smaller the equilibrium dissociation constant, the tighter the antibody-antigen binding, and the higher the affinity between the antibody and the antigen. The specific binding properties between two molecules can be measured using methods known in the art, for example, measured by surface plasmon resonance (SPR) with a BIACORE instrument.

As used herein, the detectable label of the present invention may be any substance detectable by fluorescent, spectroscopic, photochemical, biochemical, immunological, electrical, optical or chemical means. Such labels are well known in the art and examples include, but are not limited to, enzyme (e.g., horseradish peroxidase, alkaline phosphatase, β-galactosidase, urease, glucose oxidase, etc.), radionuclide (e.g., ³H, ¹²⁵I, ³⁵S, ¹⁴C, or ³²P), fluorescent dye (e.g., fluorescein isothiocyanate (FITC), fluorescein, tetramethylrhodamine isothiocyanate (TRITC), phycoerythrin (PE), Texas red, rhodamine, quantum dots or cyanine dye derivative (e.g., Cy7, Alexa 750)), luminescent substance (e.g., chemiluminescent substance, such as acridinium ester, luminol and derivative thereof, ruthenium derivative such as ruthenium terpyridyl), magnetic beads (e.g., Dynabeads^{®}), calorimetric marker such as colloidal gold or colored glass or plastic (e.g., polystyrene, polypropylene, latex, etc.) beads, and biotin that binds to avidin (e.g., streptavidin) modified with the above label.

As used herein, the term "vector" refers to a nucleic acid delivery vehicle into which a polynucleotide can be inserted. When the vector can express the protein encoded by the inserted polynucleotide, the vector is called an expression vector. The vector can be introduced into the host cell through transformation, transduction or transfection, so that the genetic material elements it carries can be expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); phage such as λ phage or M13 phage, and animal virus, etc. The animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may contain a variety of expression-controlling elements, including, but not limited to, promoter sequence, transcription initiation sequence, enhancer sequence, selection element, and reporter gene. In addition, a vector may also contain an origin of replication site.

As used herein, the term "host cell" refers to a cell that can be used to introduce a vector, which includes, but is not limited to, prokaryotic cell such as *E. coli* or *Bacillus subtilis,* fungal cell such as yeast cell or *Aspergillus,* etc, insect cell such as *S2 Drosophila* cell or Sf9, or animal cell such as fibroblast, CHO cell, COS cell, NSO cell, HeLa cell, BHK cell, HEK 293 cell or human cell.

As used herein, the term "conservative substitution" refers to an amino acid substitution that does not adversely affect or alter the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include those that replace an amino acid residue with an amino acid residue that has a similar side chain, for example, one that is physically or functionally similar to the corresponding amino acid residue (e.g., has similar size, shape, charge, chemical properties, including ability to form covalent or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al., Protein Eng. 12(10):879-884 (1999); and Burks et al. Proc. Natl Acad. Set USA 94:412-417 (1997), which is incorporated herein by reference).

The writing of the twenty conventional amino acids involved herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by one-letter and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995) and includes, but is not limited to: pH adjusting agent, surfactant, adjuvant, ionic strength enhancing agent, diluent, agent for maintaining osmotic pressure, agent for delaying absorption, preservative. For example, the pH adjusting agent includes, but is not limited to, phosphate buffer. The surfactant includes, but is not limited to, cationic, anionic or nonionic surfactant such as Tween-80. The ionic strength enhancing agent includes, but is not limited to, sodium chloride. The preservative includes, but is not limited to, various antibacterial and antifungal agents, such as paraben, chlorobutanol, phenol, sorbic acid, etc. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), etc. The preservative includes, but is not limited to, various antibacterial and antifungal agents, such as thimerosal, 2-phenoxyethanol, paraben, chlorobutanol, phenol, sorbic acid, etc. Stabilizers have the meaning generally understood by those skilled in the art, which can stabilize the desired activity of active ingredient in medicines, including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dry whey, albumin or casein) or degradation product thereof (e.g., lactalbumin hydrolyzate), etc. In certain exemplary embodiments, the pharmaceutically acceptable carrier or excipient includes sterile injectable liquid (e.g., aqueous or non-aqueous suspension or solution). In certain exemplary embodiments, such sterile injectable liquid is selected from water for injection (WFI), bacteriostatic water for injection (BWFI), sodium chloride solution (e.g., 0.9% (w/v) NaCl), dextrose solution (e.g., 5% glucose), surfactant-containing solution (e.g., solution containing 0.01% polysorbate 20), pH buffer solution (e.g., phosphate buffer solution), Ringer's solution and any combination thereof.

As used herein, the term "adjuvant" refers to a non-specific immune enhancer that, when delivered into an organism together with an antigen or in advance, can enhance the immune response to the antigen or change the type of immune response in the organism. There are many kinds of adjuvants, including but not limited to aluminum adjuvant (e.g., aluminum hydroxide), Freund's adjuvant (e.g., complete Freund's adjuvant and incomplete Freund's adjuvant), *corynebacterium parvum,* lipopolysaccharide, cytokine, etc. Freund's adjuvant is currently the most commonly used adjuvant in animal testing. Aluminum hydroxide adjuvant is commonly used in clinical trials.

As used herein, the term "prevention" refers to a method performed to prevent or delay the occurrence of a disease or disorder or symptom (e.g., HRV infection) in a subject. As used herein, the term "treatment" refers to a method performed to obtain a beneficial or desired clinical result. For the purposes of the present invention, beneficial or desired clinical result includes (but is not limited to) alleviation of symptom, reduction of the extent of disease, stabilization (i.e., no worsening) of the state of disease, delaying or slowing the progression of disease, ameliorating or alleviating the state of disease, and relief of symptom (whether partial or complete), whether detectable or undetectable. In addition, "treatment" may also refer to prolonging survival compared with the expected survival if no treatment is received.

As used herein, the term "subject" refers to a mammal, such as a human. In certain embodiments, the subject (e.g., a human) has, or is at risk for, an HRV infection or a disease associated with an HRV infection.

As used herein, the term "effective amount" refers to an amount sufficient to obtain, or at least partially obtain, the desired effect. For example, an effective amount to prevent a disease (e.g., HRV infection) refers to an amount sufficient to prevent, arrest, or delay the occurrence of a disease (e.g., an HRV infection); an effective amount to treat a disease refers to an amount sufficient to cure or at least partially prevent an existing disease or complication thereof in a patient suffering to the disease. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner in which the drug is administered, and other treatments administered concurrently, and so on.

As used herein, the term "neutralizing activity" refers to that the antibody or antibody fragment has the functional activity to bind to an antigenic protein on a virus, thereby preventing virus from infecting cells and/or the maturation of viral progeny and/or the release of viral progeny. The antibody or antibody fragment with neutralizing activity can prevent the amplification of the virus, thereby inhibiting or eliminating the viral infection.

### Brief Description of the Drawings

Fig. 1 shows the sequence conservation analysis results of polypeptides 2C01 (panel A), 2C02 (panel B) and 2C03 (panel C) in 117 virus strains.
Fig. 2 shows the SDS-PAGE electrophoresis results of fusion proteins C149-2C (aa 12-20), C149-2C (aa 151-163) C149-2C (aa 186-193), C149-2C (aa 149-165), C149-2C (aa 147-167), and C149-2C (aa 145-169).
Fig. 3 shows the electron micrographs of virus-like particles assembled by recombinant proteins C149-2C (aa 12-20), C149-2C (aa 151-163), C149-2C (aa 186-193), C149-2C (aa 149-165), C149-2C (aa 147-167), and C149-2C (aa 145-169).
Fig. 4 shows the intensity histograms of OD (450/620) of binding affinity of the screened monoclonal antibody 9A5 to polypeptides 2C01, 2C02 and 2C03 respectively, as detected by ELISA.
Fig. 5 shows the intensity histograms of OD (450/620) of binding affinity of the screened monoclonal antibody 9A5-HRP to proteins C149-2C (aa 151-163), C149-2C (aa 12-20) and C149-2C (aa 186-193) respectively, as detected by ELISA.
Fig. 6 shows the immunofluorescence image of monoclonal antibody 9A5 on H1-Hela cells infected with rhinovirus.
Fig. 7 shows the spot number of virus-infected H1-Hela cells detected by ELISPOT using monoclonal antibody 9A5.

### Sequence Information

The sequences involved in the present application are described in the table below.

**Table 1: Sequence information**

| SEQ ID NO: | Description of sequence |
|---|---|
| 1 | Amino acid sequence of polypeptide 2C01 |
| | YSLPPDPKYFDGY |
| 2 | Amino acid sequence of polypeptide 2C02 |
| | CNAAKGLEW |
| 3 | Amino acid sequence of polypeptide 2C03 |
| | FCQMVSTT |
| 4 | Amino acid sequence of HBcAg |
| | MDIDPYKEFGASVELLSFLPSDFFPSIRDLLDTASALYREALESPEHCSPHH TALRQAILCWGELMNLATWVGSNLEDPASRELVVSYVNVNMGLKIRQLL WFHISCLTFGRETVLEYLVSFGVWIRTPPAYRPQNAPILSTLPETTVVRRR CRSPRRRTPSPRRRRSQSPRRRRSQSRESQC |
| 5 | Amino acid sequence of C149-2C (aa 151-163)(C149-2C01) |
| | MDIDPYKEFGASVELLSFLPSDFFPSIRDLLDTASALYREALESPEHCSPHH TALRQAILCWGELMNLATWVGSNLEDGGGGSGGGGTGSYSLPPDPKYFD GYEFGGGGSGGGGSRELVVSYVNVNMGLKIRQLLWFHISCLTLGRETVL EYLVSFGVWIRTPPAYRPQNAPILSTLPETTVV |
| 6 | Amino acid sequence of C149-2C (aa 12-20) (C149-2C02) |
| | MDIDPYKEFGASVELLSFLPSDFFPSIRDLLDTASALYREALESPEHCSPHH TALRQAILCWGELMNLATWVGSNLEDGGGGSGGGGTGSCNAAKGLEWE FGGGGSGGGGSRELVVSYVNVNMGLKIRQLLWFHISCLTLGRETVLEYL VSFGVWIRTPPAYRPQNAPILSTLPETTVV |
| 7 | Amino acid sequence of C149-2C (aa 186-193)(C149-2C03) |
| | MDIDPYKEFGASVELLSFLPSDFFPSIRDLLDTASALYREALESPEHCSPHH TALRQAILCWGELMNLATWVGSNLEDGGGGSGGGGTGSFCQMVSTTEF GGGGSGGGGSRELVVSYVNVNMGLKIRQLLWFHISCLTLGRETVLEYLV SFGVWIRTPPAYRPQNAPILSTLPETTVV |
| 8 | Amino acid sequence of linker |
| | GGGGSGGGGTGSFEFGGGGSGGGG |
| 9 | Amino acid sequence of human rhinovirus (W10(HRV-C15)) non-structural protein 2C |
| | SDSWLRKFTECCNAAKGLEWISIKIGKFIDWLKGKLVPAVQRKRDTLDRC KKISLLEEQVNGFSSASSEAQQQLIVEVDTLKKGLDELAPLYASENKRVTK IQKDLKQLSAYLKNHRHEPVCLLLHGNPGCGKSLVTTIIARGLTQEAQVY SLPPDPKYFDGYDQQQVVILDDLGQNPDGKDLSTFCQMVSTTDFIVPMAS LEDKGKSFTSQYVLASTNLDTLSPPTVTIPEAIKRRFFLDADLITTSKFRNT TGLLDVAKALQPCTGCPKPAHYKTCCPLLCGKAVVVQDRKTKANFSVNT IVEQLRHENATRKKVKHNLDAIFQ |
| 10 | Nucleotide sequence of primer 1 for 2C (aa 151-163) |
| | GATCCTACTCACTACCACCTGATCCCAAATATTTCGATGGTTATG |
| 11 | Nucleotide sequence of primer 2 for 2C (aa 151-163) |
| | AATTCATAACCATCGAAATATTTGGGATCAGGTGGTAGTGAGTAG |
| 12 | Nucleotide sequence of primer 1 for 2C (aa 12-20) |
| | GATCCTGCAATGCAGCAAAAGGACTTGAATGGG |
| 13 | Nucleotide sequence of primer 2 for 2C (aa 12-20) |
| | AATTCCCATTCAAGTCCTTTTGCTGCATTGCAG |
| 14 | Nucleotide sequence of primer 1 for 2C (aa 186-193) |
| | GATCCTTCTGTCAGATGGTATCAACAACAG |
| 15 | Nucleotide sequence of primer 2 for 2C (aa 186-193) |
| | AATTCTGTTGTTGATACCATCTGACAGAAG |
| 16 | Nucleotide sequence of primer MVkF-G1 |
| | ACTAGTCGACATGAAGTTGCCTGTTAGGCTGTTGGTGCT |
| 17 | Nucleotide sequence of primer MVkR-M13 |
| | CCCAAGCTTACTGGATGGTGGGAAGATGGA |
| 18 | Nucleotide sequence encoding 9A5 light chain variable region (VL) |
| | GATGTTGTGATGACCCAGACTCCACTCACCTTGTCGGTTACCATTGGAC AACCAGCCTCCATCTCTTGCAAGTCAAGTCAGAGCCTCTTAGATAGTG ATGGAAAGACATATTTGAATTGGTTGTTACAGAGGCCAGGCCAGTCTC CAAAGCGCCTAATCTATCTGGTGTCTAAACTGGACTCTGGAGTCCCTG ACAGGTTCACTGGCAGTGGATCAGGGACAGATTTCACACTGAAAATCA GCAGAGTGGAGGCTGAGGATTTGGGAGTTTATTATTGCTGGCAAGGTA CACATCTTCCGCACACGTTCGGAGGGGGGACCAAGCTGGAAACAAAA CGG |
| 19 | Amino acid sequence of 9A5 light chain variable region (VL) |
| | DVVMTQTPLTLSVTIGQPASISCKSSQSLLDSDGKTYLNWLLQRPGQSPKR LIYLVSKLDSGVPDRFTGSGSGTDFTLKISRVEAEDLGVYYCWQGTHLPH TFGGGTKLETKR |
| 20 | Nucleotide sequence of primer MVhF-C1 |
| | ACTAGTCGACATGGACTCCAGGCTCAATTTAGTTTTCCT |
| 21 | Nucleotide sequence of primer MVhR-M13 |
| | CCCAAGCTTCCAGGGRCCARKGGATARACGRTGG |
| 22 | Nucleotide sequence encoding 9A5 heavy chain variable region (VH) |
| | GAGGTGCAGCTGGTGGAGTCTGGGGGAGACTTAGTGAAGCCTGGAGG GTCCCTGAAACTCTCCTGTGCAGCCTCTGGATTCACTTTCAGTATCTAT GGCATGTCTTGGGTTCGCCAGACTCCAGACAAGAGACTGGAGTGGGTC GCAACCATTAGTAGTGCTGGTATTTACACCTACTATCCAGACAGTGTG AAGGGGCGATTCACCATCTCCAGAGACAATGCCAAGAACACCCTGTAC CTGCAAATGAGCAGTCTGAAGTCTGAGGACACAGCCATGTATTACTGT GCAAGACACGGCTACGGGTTGTTCGATGTCTGGGGCGCAGGGACCAC GGTCACCGTCTCCTCA |
| 23 | Amino acid sequence of 9A5 heavy chain variable region (VH) |
| | EVQLVESGGDLVKPGGSLKLSCAASGFTFSIYGMSWVRQTPDKRLEWVA TISSAGIYTYYPDSVKGRFTISRDNAKNTLYLQMSSLKSEDTAMYYCARH GYGLFDVWGAGTTVTVSS |
| 24 | Amino acid sequence of VL CDR1 |
| | QSLLDSDGKTY |
| 25 | Amino acid sequence of VL CDR2 |
| | LVS |
| 26 | Amino acid sequence of VL CDR3 |
| | WQGTHLPHT |
| 27 | Amino acid sequence of VH CDR1 |
| | GFTFSIYG |
| 28 | Amino acid sequence of VH CDR2 |
| | ISSAGIYT |
| 29 | Amino acid sequence of VH CDR3 |
| | ARHGYGLFDV |
| 30 | Amino acid sequence of rhinovirus non-structural protein 2C aa 151-163 (2C04) |
| | YSLPPDPKHFDGY |
| 31 | Amino acid sequence of rhinovirus non-structural protein 2C aa 151-163 (2C05) |
| | YSLPPAPKYFDGY |
| 32 | Amino acid sequence of rhinovirus non-structural protein 2Caa 151-163 (2C06) |
| | YSLPPSPKYFDGY |
| 33 | Amino acid sequence of rhinovirus non-structural protein 2C aa 151-163 (2C07) |
| | YSLPPNPKHFDGY |
| 34 | Amino acid sequence of rhinovirus non-structural protein 2C aa 151-163 (2C08) |
| | YSLPPNPKYFDGY |
| 35 | Amino acid sequence of rhinovirus non-structural protein 2C aa 149-165 (2C09) |
| | QVYSLPPDPKYFDGYDQ |
| 36 | Amino acid sequence of rhinovirus non-structural protein 2C aa 147-167 (2C10) |
| | EAQVYSLPPDPKYFDGYDQQQ |
| 37 | Amino acid sequence of rhinovirus non-structural protein 2C aa 145-169 (2C11) |
| | TQEAQVYSLPPDPKYFDGYDQQQVV |
| 38 | Nucleotide sequence of primer 1 for 2C (aa 149-165) |
| | GATCCCAGGTATACTCACTACCACCTgATCCCAAATATTTCGATGGTTA TGACCAAG |
| 39 | Nucleotide sequence of primer 2 for 2C (aa 149-165) |
| | AATTCTTGGTCATAACCATCGAAATATTTGGGATCAGGTGGTAGTGAG TATACCTGG |
| 40 | Nucleotide sequence of primer 1 for 2C (aa 147-167) |
| | GATCCGAGGCACAGGTATACTCACTACCACCTGATCCCAAATATTTCG ATGGTTATGACCAACAGCAGG |
| 41 | Nucleotide sequence of primer 2 for 2C (aa 147-167) |
| | AATTCCTGCTGTTGGTCATAACCATCGAAATATTTGGGATCAGGTGGT AGTGAGTATACCTGTGCCTCG |
| 42 | Nucleotide sequence of primer 1 for 2C (aa 145-169) |
| | GATCCACCCAGGAGGCACAGGTATACTCACTACCACCTGATCCCAAAT ATTTCGATGGTTATGACCAACAGCAGGTTGTCGG |
| 43 | Nucleotide sequence of primer 2 for 2C (aa 145-169) |
| | AATTCGACAACCTGCTGTTGGTCATAACCATCGAAATATTTGGGATCA GGTGGTAGTGAGTATACCTGTGCCTCCTGGGTG |
| 44 | Amino acid sequence of C149-2C (aa 151-163)(C149-2C04) |
| | MDIDPYKEFGASVELLSFLPSDFFPSIRDLLDTASALYREALESPEHCSPHH TALRQAILCWGELMNLATWVGSNLEDGGGGSGGGGTGSYSLPPDPKHFD GYEFGGGGSGGGGSRELVVSYVNVNMGLKIRQLLWFHISCLTLGRETVL EYLVSFGVWIRTPPAYRPQNAPILSTLPETTVV |
| 45 | Amino acid sequence of C149-2C (aa 151-163) (C149-2C05) |
| | MDIDPYKEFGASVELLSFLPSDFFPSIRDLLDTASALYREALESPEHCSPHH TALRQAILCWGELMNLATWVGSNLEDGGGGSGGGGTGSYSLPPAPKYFD GYEFGGGGSGGGGSRELVVSYVNVNMGLKIRQLLWFHISCLTLGRETVL EYLVSFGVWIRTPPAYRPQNAPILSTLPETTVV |
| 46 | Amino acid sequence of C149-2C (aa 151-163) (C149-2C06) |
| | MDIDPYKEFGASVELLSFLPSDFFPSIRDLLDTASALYREALESPEHCSPHH TALRQAILCWGELMNLATWVGSNLEDGGGGSGGGGTGSYSLPPSPKYFD GYEFGGGGSGGGGSRELVVSYVNVNMGLKIRQLLWFHISCLTLGRETVL EYLVSFGVWIRTPPAYRPQNAPILSTLPETTVV |
| 47 | Amino acid seauence of C149-2C (aa 151-163)(C149-2C07) |
| | MDIDPYKEFGASVELLSFLPSDFFPSIRDLLDTASALYREALESPEHCSPHH TALRQAILCWGELMNLATWVGSNLEDGGGGSGGGGTGSYSLPPNPKHFD GYEFGGGGSGGGGSRELVVSYVNVNMGLKIRQLLWFHISCLTLGRETVL EYLVSFGVWIRTPPAYRPQNAPILSTLPETTVV |
| 48 | Amino acid sequence of C149-2C (aa 151-163) (C149-2C08) |
| | MDIDPYKEFGASVELLSFLPSDFFPSIRDLLDTASALYREALESPEHCSPHH TALRQAILCWGELMNLATWVGSNLEDGGGGSGGGGTGSYSLPPNPKYFD GYEFGGGGSGGGGSRELVVSYVNVNMGLKIRQLLWFHISCLTLGRETVL EYLVSFGVWIRTPPAYRPQNAPILSTLPETTVV |
| 49 | Amino acid sequence of C149-2C (aa 149-165) (C149-2C09) |
| | MDIDPYKEFGASVELLSFLPSDFFPSIRDLLDTASALYREALESPEHCSPHH TALRQAILCWGELMNLATWVGSNLEDGGGGSGGGGTGSQVYSLPPDPK YFDGYDQEFGGGGSGGGGSRELVVSYVNVNMGLKIRQLLWFHISCLTLG |
| | RETVLEYLVSFGVWIRTPPAYRPQNAPILSTLPETTVV |
| 50 | Amino acid sequence of C149-2C (aa 147-167) (C149-2C10) |
| | MDIDPYKEFGASVELLSFLPSDFFPSIRDLLDTASALYREALESPEHCSPHH TALRQAILCWGELMNLATWVGSNLEDGGGGSGGGGTGSEAQVYSLPPDP KYFDGYDQQQEFGGGGSGGGGSRELVVSYVNVNMGLKIRQLLWFHISCL TLGRETVLEYLVSFGVWIRTPPAYRPQNAPILSTLPETTVV |
| 51 | Amino acid sequence of C149-2C (aa 145-169) (C149-2C11) |
| | MDIDPYKEFGASVELLSFLPSDFFPSIRDLLDTASALYREALESPEHCSPHH TALRQAILCWGELMNLATWVGSNLEDGGGGSGGGGTGSTQEAQVYSLPP DPKYFDGYDQQQVVEFGGGGSGGGGSRELVVSYVNVNMGLKIRQLLWF HISCLTLGRETVLEYLVSFGVWIRTPPAYRPQNAPILSTLPETTVV |
| 52 | formula of C149-2C (aa 151-163) |
| | YSLPPX₁PKX₂FDGY |

| | |
|---|---|
| Note: R=A or G; K=G or T | |

### Deposit information of biological material

The present invention relates to the following biological material that has been deposited in the China Center for Type Culture Collection (CCTCC, Wuhan University, Wuhan, China):
Hybridoma cell strain 9A5: its deposit number is CCTCC No. C2021141, its deposit date is May 28, 2021, and its deposit address is Luojiashan, Wuchang, Wuhan City (Postal Code 430072).

### Specific Models for Carrying Out the present invention

The embodiments of the present invention will be described in detail below with reference to examples. Those skilled in the art will understand that the following examples are only used to illustrate the present invention and should not be regarded as limiting the scope of the present invention. If the specific techniques or conditions were not specified in the examples, the techniques or conditions described in the literature in the field (e.g., "Molecular Cloning Experimental Guide" J. Sambrook et al., translated by Huang Peitang et al., third edition, Science Press) or the product instructions were used. If the manufacturer of the reagents or instruments used was not indicated, they were all conventional products purchased commercially.

### Example 1: Design of highly conserved polypeptide of human rhinovirus

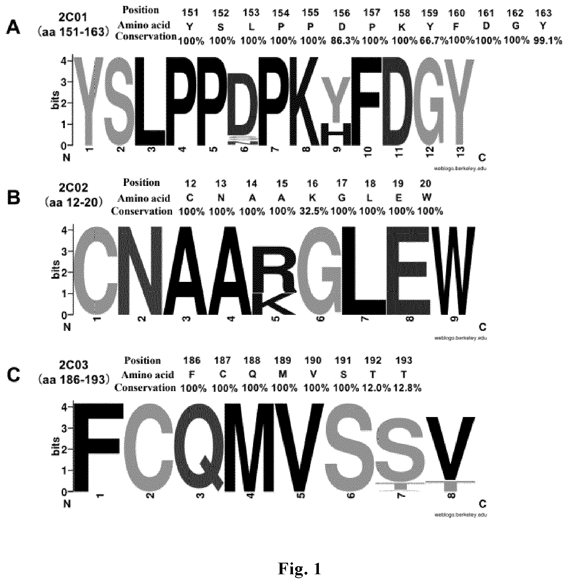

In this example, Clustal software was used to perform homology analysis on the human rhinovirus protein 2C amino acid sequences of different serotypes. The strain sequences used included a total of 117 strains of viruses, including HRV-A1B, HRV-A2, HRV-B14, HRV-C15, etc., and the specific information of the strains was shown in Table 2. After homology analysis, three highly conserved peptide segments located in human rhinovirus protein 2C were selected: peptide 2C01 (aa 151-163): YSLPPDPKYFDGY (SEQ ID NO: 1), peptide 2C02 (aa 12-20): CNAAKGLEW (SEQ ID NO: 2), and peptide 2C03 (aa 186-193): FCQMVSTT (SEQ ID NO: 3). The amino acid sequences of these three peptides (with 13, 9 and 8 amino acids in length, respectively) all had high conservation and homology among the total 117 strains of viruses. The sequence conservation analysis results of the three polypeptides were shown in Fig. 1.

**Table 2. Information of virus strains selected for amino acid sequence alignment**

| Type | GenBank No. | Type | GenBank No. | Type | GenBank No. |
|---|---|---|---|---|---|
| HRV-A1B | D00239 | HRV-A54 | FJ445138 | HRV-B17 | EF173420 |
| HRV-A2 | X02316 | HRV-A55 | DQ473511 | HRV-B26 | FJ445124 |
| HRV-A7 | DQ473503 | HRV-A56 | FJ445140 | HRV-B27 | EF173421 |
| HRV-A8 | FJ445113 | HRV-A57 | KY369874 | HRV-B35 | MN369044 |
| HRV-A9 | FJ445177 | HRV-A58 | FJ445142 | HRV-B37 | EF173423 |
| HRV-A10 | DQ473498 | HRV-A59 | DQ473500 | HRV-B42 | FJ445130 |
| HRV-A11 | EF173414 | HRV-A60 | FJ445143 | HRV-B48 | DQ473488 |
| HRV-A12 | EF173415 | HRV-A61 | KY369886 | HRV-B52 | EF173424 |
| HRV-A13 | FJ445117 | HRV-A62 | FJ445145 | HRV-B69 | FJ445151 |
| HRV-A15 | DQ473493 | HRV-A63 | FJ445146 | HRV-B70 | DQ473489 |
| HRV-A16 | JN562722 | HRV-A64 | EF173417 | HRV-B72 | JN614997 |
| HRV-A18 | JF781496 | HRV-A65 | FJ445147 | HRV-B79 | FJ445155 |
| HRV-A19 | FJ445119 | HRV-A66 | FJ445148 | HRV-B83 | FJ445161 |
| HRV-A20 | FJ445120 | HRV-A67 | FJ445149 | HRV-B84 | JF781499 |
| HRV-A21 | MF043119 | HRV-A68 | FJ445150 | HRV-B86 | FJ445164 |
| HRV-A22 | KY369885 | HRV-A73 | DQ473492 | HRV-B91 | FJ445168 |
| HRV-A23 | DQ473497 | HRV-A74 | DQ473494 | HRV-B92 | FJ445169 |
| HRV-A24 | EF173416 | HRV-A75 | DQ473510 | HRV-B93 | EF173425 |
| HRV-A25 | FJ445123 | HRV-A76 | DQ473502 | HRV-B97 | KY369883 |
| HRV-A29 | FJ445125 | HRV-A77 | FJ445154 | HRV-B99 | FJ445174 |
| HRV-A30 | DQ473512 | HRV-A78 | EF173418 | HRV-C2 | KY369881 |
| HRV-A31 | KY369884 | HRV-A80 | FJ445156 | HRV-C3 | MN228693 |
| HRV-A32 | FJ445127 | HRV-A81 | FJ445159 | HRV-C6 | JN990702 |
| HRV-A33 | FJ445128 | HRV-A82 | KY369894 | HRV-C11 | KY369877 |
| HRV-A34 | DQ473501 | HRV-A85 | FJ445163 | HRV-C15 | GU219984 |
| HRV-A36 | DQ473505 | HRV-A88 | DQ473504 | HRV-C23 | KJ675506 |
| HRV-A38 | DQ473495 | HRV-A89 | FJ445166 | HRV-C24 | EF582385 |
| HRV-A39 | KT726984 | HRV-A90 | FJ445167 | HRV-C26 | EF582387 |
| HRV-A40 | MK167031 | HRV-A94 | EF173419 | HRV-C32 | MK520815 |
| HRV-A41 | MN369037 | HRV-A95 | FJ445170 | HRV-C39 | JN205461 |
| HRV-A43 | FJ445131 | HRV-A96 | FJ445171 | HRV-C41 | MK279354 |
| HRV-A44 | DQ473499 | HRV-A98 | KY369875 | HRV-C42 | MH752985 |
| HRV-A45 | FJ445132 | HRV-A100 | FJ445175 | HRV-C43 | JX074056 |
| HRV-A46 | DQ473506 | HRV-A101 | GQ415051 | HRV-C45 | JN837686 |
| HRV-A47 | FJ445133 | HRV-B3 | DQ473485 | HRV-C47 | MF806525 |
| HRV-A49 | DQ473496 | HRV-B4 | DQ473490 | HRV-C49 | JF907574 |
| HRV-A50 | FJ445135 | HRV-B5 | FJ445112 | HRV-C51 | JF317015 |
| HRV-A51 | FJ445136 | HRV-B6 | DQ473486 | HRV-C53 | MF775366 |
| HRV-A53 | DQ473507 | HRV-B14 | K02121 | HRV-C56 | MG950178 |

The human rhinovirus non-structural protein 2C aa 151-163 amino acid sequences of the above 117 virus strains were provided in Table 3 (SEQ ID NOs: 1, 30 to 34), respectively.

**Table 3. Amino acid sequences of non-structural protein 2C aa 151-163 of 117 virus strains**

| Type | Amino acid sequence (SEQ ID NO:) | Type | Amino acid sequence (SEQ ID NO:) | Type | Amino acid sequence (SEQ ID NO:) |
|---|---|---|---|---|---|
| HRV-A1 B | YSLPPDPKYFD GY(1) | HRV-A54 | YSLPPDPKYF DGY(1) | HRV-B17 | YSLPPDPKHF DGY(30) |
| HRV-A2 | YSLPPDPKYFD GY(1) | HRV-A55 | YSLPPDPKYF DGY(1) | HRV-B26 | YSLPPDPKHF DGY(30) |
| HRV-A7 | YSLPPDPKYFD GY(1) | HRV-A56 | YSLPPDPKYF DGY(1) | HRV-B27 | YSLPPDPKHF DGY(30) |
| HRV-A8 | YSLPPDPKYFD GY(1) | HRV-A57 | YSLPPDPKYF DGY(1) | HRV-B35 | YSLPPDPKHF DGY(30) |
| HRV-A9 | YSLPPDPKYFD GY(1) | HRV-A58 | YSLPPDPKYF DGY(1) | HRV-B37 | YSLPPDPKHF DGY(30) |
| HRV-A1 0 | YSLPPDPKYFD GY(1) | HRV-A59 | YSLPPDPKYF DGY(1) | HRV-B42 | YSLPPDPKHF DGY(30) |
| HRV-A1 1 | YSLPPDPKYFD GY(1) | HRV-A60 | YSLPPDPKYF DGY(1) | HRV-B48 | YSLPPDPKHF DGY(30) |
| HRV-A1 2 | YSLPPAPKYFD GY(31) | HRV-A61 | YSLPPDPKYF DGY(1) | HRV-B52 | YSLPPDPKHF DGY(30) |
| HRV-A1 3 | YSLPPDPKYFD GY(1) | HRV-A62 | YSLPPDPKYF DGY(1) | HRV-B69 | YSLPPDPKHF DGY(30) |
| HRV-A1 5 | YSLPPDPKYFD GY(1) | HRV-A63 | YSLPPDPKYF DGY(1) | HRV-B70 | YSLPPDPKHF DGY(30) |
| HRV-A1 6 | YSLPPDPKYFD GY(1) | HRV-A64 | YSLPPDPKYF DGY(1) | HRV-B72 | YSLPPDPKHF DGY(30) |
| HRV-A1 8 | YSLPPDPKYFD GY(1) | HRV-A65 | YSLPPSPKYFD GY(32) | HRV-B79 | YSLPPDPKHF DGY(30) |
| HRV-A1 9 | YSLPPDPKYFD GY(1) | HRV-A66 | YSLPPDPKYF DGY(1) | HRV-B83 | YSLPPDPKHF DGY(30) |
| HRV-A2 0 | YSLPPSPKYFD GY(32) | HRV-A67 | YSLPPDPKYF DGY(1) | HRV-B84 | YSLPPDPKHF DGY(30) |
| HRV-A2 1 | YSLPPDPKYFD GY(1) | HRV-A68 | YSLPPSPKYFD GY(32) | HRV-B86 | YSLPPDPKHF DGY(30) |
| HRV-A2 2 | YSLPPDPKYFD GY(1) | HRV-A73 | YSLPPDPKYF DGY(1) | HRV-B91 | YSLPPDPKHF DGY(30) |
| HRV-A2 3 | YSLPPDPKYFD GY(1) | HRV-A74 | YSLPPDPKYF DGY(1) | HRV-B92 | YSLPPDPKHF DGY(30) |
| HRV-A2 | YSLPPDPKYFD | HRV-A75 | YSLPPDPKYF | HRV-B93 | YSLPPDPKHF |
| 4 | GY(1) | | DGY (1) | | DGY(30) |
| HRV-A2 5 | YSLPPDPKYFD GY(1) | HRV-A76 | YSLPPDPKYF DGY(1) | HRV-B97 | YSLPPDPKHF DGY(30) |
| HRV-A2 9 | YSLPPDPKYFD GY(1) | HRV-A77 | YSLPPDPKYF DGY(1) | HRV-B99 | YSLPPDPKHF DGY(30) |
| HRV-A3 0 | YSLPPDPKYFD GY(1) | HRV-A78 | YSLPPDPKHF DGY(30) | HRV-C2 | YSLPPNPKHF DGY(33) |
| HRV-A3 1 | YSLPPDPKYFD GY(1) | HRV-A80 | YSLPPSPKYFD GY(32) | HRV-C3 | YSLPPDPKYF DGY(1) |
| HRV-A3 2 | YSLPPDPKYFD GY(1) | HRV-A81 | YSLPPDPKYF DGY(1) | HRV-C6 | YSLPPDPKYF DGY(1) |
| HRV-A3 3 | YSLPPDPKYFD GY(1) | HRV-A82 | YSLPPDPKYF DGY(1) | HRV-C11 | YSLPPNPKHF DGY(33) |
| HRV-A3 4 | YSLPPDPKYFD GY(1) | HRV-A85 | YSLPPDPKYF DGY(1) | HRV-C15 | YSLPPDPKYF DGY(1) |
| HRV-A3 6 | YSLPPDPKYFD GY(1) | HRV-A88 | YSLPPDPKYF DGY(1) | HRV-C23 | YSLPPDPKHF DGY(30) |
| HRV-A3 8 | YSLPPDPKYFD GY(1) | HRV-A89 | YSLPPDPKYF DGY(1) | HRV-C24 | YSLPPDPKHF DGY(30) |
| HRV-A3 9 | YSLPPDPKYFD GY(1) | HRV-A90 | YSLPPDPKYF DGY(1) | HRV-C26 | YSLPPDPKYF DGY(1) |
| HRV-A4 0 | YSLPPDPKYFD GY(1) | HRV-A94 | YSLPPDPKYF DGY(1) | HRV-C32 | YSLPPDPKHF DGY(30) |
| HRV-A4 1 | YSLPPDPKYFD GY(1) | HRV-A95 | YSLPPDPKYF DGY(1) | HRV-C39 | YSLPPDPKHF DGY(30) |
| HRV-A4 3 | YSLPPDPKYFD GY(1) | HRV-A96 | YSLPPDPKYF DGY(1) | HRV-C41 | YSLPPDPKHF DGY(30) |
| HRV-A4 4 | YSLPPDPKYFD GY(1) | HRV-A98 | YSLPPDPKYF DGY(1) | HRV-C42 | YSLPPDPKYF DGY(1) |
| HRV-A4 5 | YSLPPNPKYFD GY(34) | HRV-A10 0 | YSLPPDPKYF DGY(1) | HRV-C43 | YSLPPDPKYF DGY(1) |
| HRV-A4 6 | YSLPPSPKYFD GY(32) | HRV-A10 1 | YSLPPSPKYFD GY(32) | HRV-C45 | YSLPPNPKHF DGY(33) |
| HRV-A4 7 | YSLPPDPKYFD GY(1) | HRV-B3 | YSLPPDPKYF DGY(1) | HRV-C47 | YSLPPNPKHF DGY(33) |
| HRV-A4 9 | YSLPPDPKYFD GY(1) | HRV-B4 | YSLPPDPKHF DGY(30) | HRV-C49 | YSLPPDPKHF DGY(30) |
| HRV-A5 0 | YSLPPDPKYFD GY(1) | HRV-B5 | YSLPPDPKHF DGY(30) | HRV-C51 | YSLPPNPKHF DGY(33) |
| HRV-A5 1 | YSLPPSPKYFD GY(32) | HRV-B6 | YSLPPDPKHF DGY(30) | HRV-C53 | YSLPPNPKHF DGY(33) |
| HRV-A5 3 | YSLPPSPKYFD GY(32) | HRV-B14 | YSLPPDPKHF DGY(30) | HRV-C56 | YSLPPDPKHF DGY(30) |

### Example 2: Preparation of antiserum induced by human rhinovirus highly conserved polypeptide

Based on the amino acid sequences of the three peptides screened in Example 1, three polypeptides were designed and synthesized (synthesized by Genscript Biotechnology Co., Ltd.), and numbered as: 2C01 (hereinafter also referred to as 2C (aa 151-163), SEQ ID NO:1), 2C02 (hereinafter also referred to as 2C (aa 12-20), SEQ ID NO.2) and 2C03 (hereinafter also referred to as 2C (aa 186-193), SEQ ID NO:3). The synthesized polypeptides were used to immunize 6-week-old BALB/c female mice. Each polypeptide was used to immunize 5 mice in parallel (numbered as: Mouse 1 to Mouse 5). The immunization dose was 100 µg/mouse. In the primary immunization, Freund's complete adjuvant (purchased from Sigma, product number: F5881) was mixed with the polypeptide, then booster immunization was carried out every two weeks and Freund's incomplete adjuvant (purchased from Sigma, product number: F5881) was mixed with the polypeptide in the booster immunization, there were a total of three booster shots, and blood samples were collected on the 14th day after the third booster shot.

### Example 3: Preparation of antiserum induced by the variant of human rhinovirus highly conserved polypeptide

In this example, based on the amino acid sequences of non-structural protein 2C aa 151-163 of 117 human rhinovirus strains, five variant peptides with different mutations and three variant peptides with extended length were obtained by supplementary design and synthesis (synthesized by Genscript Biotechnology Co., Ltd.): i.e., polypeptide variant 2C04 (aa 151-163, Y159H): YSLPPDPKHFDGY (SEQ ID NO: 30); polypeptide variant 2C05 (aa151-163, D156A): YSLPPAPKYFDGY (SEQ ID NO: 31); polypeptide variant 2C06 (aa151-163, D156S): YSLPPSPKYFDGY (SEQ ID NO: 32); polypeptide variant 2C07 (aa151-163, D156N&Y159H): YSLPPNPKHFDGY (SEQ ID NO: 33); polypeptide variant 2C08 (aa151-163, D156N): YSLPPNPKYFDGY (SEQ ID NO: 34); peptide variant 2C09 (aa149-165): QVYSLPPDPKYFDGYDQ (SEQ ID NO: 35); polypeptide variant 2C10 (aa147-167): EAQVYSLPPDPKYFDGYDQQQ (SEQ ID NO: 36); and polypeptide variant 2C11 (aa145-169): TQEAQVYSLPPDPKYFDGYDQQQVV (SEQ ID NO: 37). The 8 synthesized polypeptide variants were used to immunize 6-week-old BALB/c female mice. Each polypeptide was used to immunize 5 mice in parallel (numbered as: Mouse 1 to Mouse 5). The immunization dose was 100 µg/mouse. In the primary immunization, Freund's complete adjuvant (purchased from Sigma, product number: F5881) was mixed with the polypeptide, then booster immunization was carried out every two weeks and Freund's incomplete adjuvant (purchased from Sigma, product number: F5881) was mixed with the polypeptide in the booster immunization, there were a total of three booster shots, and blood samples were collected on the 14th day after the third booster shot.

### Example 4: Identification of binding activity of antiserum induced by human rhinovirus highly conserved polypeptide to human rhinovirus

The polypeptide-induced antisera obtained in Example 2 and Example 3 were tested against different representative strains of human rhinovirus groups A, B and C using the indirect immunofluorescence method. The representative human rhinovirus strains belong to the following types, respectively: Group A type 1B (HRV-A1B), Group A type 2 (HRV-A2), Group A type 9 (HRV-A9), Group A type 16 (HRV- A16), Group A type 21 (HRV-A21), Group A type 23 (HRV-A23), Group A type 29 (HRV-A29), Group A type 34 (HRV-A34), Group A type 36 (HRV- A36), Group A type 40 (HRV-A40), Group A type 43 (HRV-A43), Group A type 49 (HRV-A49), Group A type 54 (HRV-A54), Group A type 59 (HRV- A59), Group A type 77 (HRV-A77), Group A type 78 (HRV-A78), Group A type 89 (HRV-A89), Group B type 5 (HRV-B5), Group B type 6 (HRV- B6), Group B type 14 (HRV-B14), Group B type 17 (HRV-B17), Group C type 11 (HRV-C11), Group C type 15 (HRV-C15) and Group C type 23 (HRV-C23). The GenBank accession numbers of the above 24 representative human rhinoviruses were shown in Table 4. Among them, HRV-C11, HRV-C15 and HRV-C23 were obtained by our laboratory through the construction of infectious clones and virus rescue, and the rest were purchased from ATCC.

**Table 4: GenBank accession numbers of viruses**

| Type | GenBank accession number | Type | GenBank accession number |
|---|---|---|---|
| HRV-A1B | D00239 | HRV-A54 | AB079190 |
| HRV-A2 | AB079139 | HRV-A59 | AB079195 |
| HRV-A9 | AB079146 | HRV-A77 | FJ445154 |
| HRV-A16 | AB079152 | HRV-A89 | M16248 |
| HRV-A21 | AB079157 | HRV-A78 | FJ445183 |
| HRV-A23 | AB079159 | HRV-B5 | AB079142 |
| HRV-A29 | AB079165 | HRV-B6 | AB079143 |
| HRV-A34 | AB079170 | HRV-B14 | EU870450 |
| HRV-A36 | AB079172 | HRV-B17 | EF173420 |
| HRV-A40 | FJ445129 | HRV-C 11 | KY369877 |
| HRV-A43 | FJ445131 | HRV-C15 | GU219984 |
| HRV-A49 | AB079185 | HRV-C23 | KJ675506 |

The steps of immunofluorescence method were as follows:
H1-Hela cells (purchased from ATCC) cultured in DMEM (purchased from GIBCO) containing 10% FBS (purchased from ABW) were plated in a 24-well cell culture plate at a density of 1×10⁵ cells/well; after the cells adhered to the wall, each well was infected with 10³ TCID₅₀ human rhinovirus; after 20 hours, the cell culture supernatant was discarded, 500 µL of fixative solution (PBS solution containing 4% paraformaldehyde) was added to each well for fixation, and the cells were allowed to stand at room temperature in dark place for 30 minutes; after 30 minutes, the fixative solution was discarded by pipetting, 500 µL of 0.3% Triton X-100 solution was added to each well for the permeation of cells, and allowed to stand at room temperature for 10 minutes; after 10 minutes, the solution for permeation was discarded by pipetting, the cells were washed three times with PBS solution, and then the PBS solution was discarded by pipetting; an appropriate amount of blocking solution (PBS solution containing 2% BSA) was added for blocking, and allowed to stand at 37°C for reaction for 1 hour; after 1 hour, the blocking solution was discarded by pipetting, and polypeptide-induced antiserum diluted at 1:100 was added, and allowed to stand at 37°C for reaction for 1 hour; after 1 hour, the serum was discarded by pipetting, the cells were washed three times with PBS solution, and then the PBS solution was discarded by pipetting; FITC-conjugated goat anti-mouse antibody diluted at 1:500 was added, and allowed to stand at 37°C for reaction for 30 minutes; after 30 minutes, the antibody was discarded by pipetting, the cells were washed three times with PBS solution, and then the PBS solution was discarded by pipetting; DAPI dye solution was added, diluted to 1:2000, and allowed to stand at room temperature in the dark for 5 minutes; after 5 minutes, the DAPI dye solution was discarded by pipetting, the cells were washed three times with PBS solution, and then the PBS solution was discarded by pipetting; the results were obtained by observation under a fluorescence microscope after sealing. The results were shown in Table 5.

**Table 5: Binding activity of polypeptide-induced antisera to H1-Hela cells infected with rhinovirus detected by immunofluorescence**

| | 2C01 | 2C02 | 2C03 | 2C04 | 2C05 | 2C06 | 2C07 | 2C08 | 2C09 | 2C10 | 2C11 | PBS |
|---|---|---|---|---|---|---|---|---|---|---|---|---|
| HRV-A1B | + | - | - | + | + | + | + | + | + | + | + | - |
| HRV-A2 | + | - | - | + | + | + | + | + | + | + | + | - |
| HRV-A9 | + | - | - | + | + | + | + | + | + | + | + | - |
| HRV-A16 | + | - | - | + | + | + | + | + | + | + | + | - |
| HRV-A21 | + | - | - | + | + | + | + | + | + | + | + | - |
| HRV-A23 | + | - | - | + | + | + | + | + | + | + | + | - |
| HRV-A29 | + | - | - | + | + | + | + | + | + | + | + | - |
| HRV-A34 | + | - | - | + | + | + | + | + | + | + | + | - |
| HRV-A36 | + | - | - | + | + | + | + | + | + | + | + | - |
| HRV-A40 | + | - | - | + | + | + | + | + | + | + | + | - |
| HRV-A43 | + | - | - | + | + | + | + | + | + | + | + | - |
| HRV-A49 | + | - | - | + | + | + | + | + | + | + | + | - |
| HRV-A54 | + | - | - | + | + | + | + | + | + | + | + | - |
| HRV-A59 | + | - | - | + | + | + | + | + | + | + | + | - |
| HRV-A77 | + | - | - | + | + | + | + | + | + | + | + | - |
| HRV-A78 | + | - | - | + | + | + | + | + | + | + | + | - |
| HRV-A89 | + | - | - | + | + | + | + | + | + | + | + | - |
| HRV-B5 | + | - | - | + | + | + | + | + | + | + | + | - |
| HRV-B6 | + | - | - | + | + | + | + | + | + | + | + | - |
| HRV-B14 | + | - | - | + | + | + | + | + | + | + | + | - |
| HRV-B17 | + | - | - | + | + | + | + | + | + | + | + | - |
| HRV-C11 | + | - | - | + | + | + | + | + | + | + | + | - |
| HRV-C15 | + | + | + | + | + | + | + | + | + | + | + | - |
| HRV-C23 | + | - | - | + | + | + | + | + | + | + | + | - |

| | | | | | | | | | | | | |
|---|---|---|---|---|---|---|---|---|---|---|---|---|
| Note: +, positive; -, negative. | | | | | | | | | | | | |

The results in Table 5 showed that the antisera induced by polypeptide 2C01 or polypeptide variants 2C04 to 2C11 all could cross-bind to H1-Hela cells infected with 24 representative human rhinoviruses, and showed positive green fluorescence; while the antisera induced by polypeptide 2C02 or 2C03 could not cross-bind to H1-Hela cells infected with 24 representative human rhinoviruses, and showed no green fluorescence. This result showed that: (1) the immune sera induced by polypeptide 2C01 ( polypeptide 2C (aa 151-163)) or peptide variants thereof ( 2C04 (aa 151-163, Y159H), 2C05 (aa 151-163, D156A), peptide variants 2C06 (aa 151-163, D156S), peptide variant 2C07 (aa 151-163, D156N&Y159H), peptide variant 2C08 (aa 151-163, D156N), peptide variant 2C09 (aa 149-165), peptide variant 2C10 (aa 147-167), polypeptide variant 2C11 (aa 145-169)) could specifically cross-bind to human rhinovirus of different subtypes; polypeptide 2C01 and polypeptide variants thereof could contain universal affinity epitopes; (2) the sera induced by polypeptide 2C02 or 2C03 could not specifically cross-bind to human rhinoviruses of different subtypes; polypeptides 2C02 and 2C03 did not contain universal affinity epitopes. These results further indicated that not all conserved peptides contained universal affinity epitopes. On the contrary, in many cases, conserved peptides (e.g., 2C02 and 2C03) could not be used as universal affinity epitopes and could not induce the production of universal affinity antibodies.

### Example 5: Use of antiserum induced by polypeptide 2C01 to detect infection and titer of human rhinovirus

When dilution was sufficient, after rhinovirus infected cells, the enzyme-linked immunospot method could be used to make the infected cells have signals that could be detected by an Elispot instrument. The number of positive cells with signals, that was, the number of infected cells, could be considered to be the infectious units of rhinovirus that were used in this infection experiment, so that the titer of rhinovirus could be detected by the gradient dilution spot counting method. In this example, three representative rhinoviruses (HRV-A2, HRV-B14, and HRV-C15) in Example 4 were selected for the measurement of infection titer. Specific steps were as follows:
H1-Hela cells were plated in a 96-well cell culture plate at 1 × 10⁴ cells/well, and the culture medium was DMEM medium containing 2% FBS. Incubation was carried out at 33°C for 10 hours. A portion of the virus culture stock solution was taken and serially diluted 5 times in 5 gradients with serum-free DMEM medium, so that 6 concentration gradients of diluted virus solutions containing 100 µL, 20 µL, 4 µL, 0.8 µL, 0.16 µL, and 0.032 µL of virus culture stock solution per 100 µL of medium were finally obtained, respectively. 100 µL of the diluted virus solution of each gradient was taken and added to H1-Hela cells pre-plated in a 96-well cell culture plate, and 3 repeat wells were used for each gradient. The 96-well plate was cultured at 33°C. After 14 to 24 hours of culture, the detection was performed by enzyme-linked immunospot method:
(1) The culture medium was removed from each well by pipetting, 100 µL of fixative solution (PBS solution containing 0.5% formaldehyde) was added to each well, and allowed to stand in dark place for 1 hour at room temperature;
(2) After 1 hour, the fixative solution was discarded by pipetting, 100 µL of 1% Triton X-100 solution was added to each well for permeabilizing the cells, and allowed to stand at room temperature for 0.5 hours;
(3) After 0.5 h, the permeabilization solution was discarded by pipetting, the cells were washed three times with PBST solution, and then the PBST solution was discarded by pipetting;
(4) 100 µL of the antiserum induced by polypeptide 2C01 prepared in Example 2 was added to each well, the polypeptide-induced antiserum was diluted 3000 times with enzyme diluent (a buffer solution with composition of: 20 mM PBS + 0.5% casein + 2% gelatin + 0.1% preservative, the same below) before use, and the reaction was carried out at 37°C for 1 h.
(5) After 1 hour, the serum was discarded by pipetting, the cells were washed three times with PBST solution, and then the PBST solution was discarded by pipetting;
(6) 100 µL of HRP-conjugated goat anti-mouse antibody diluted at 1:5000 was added to each well, and allowed to stand at 37°C for reaction for 30 minutes;
(7) After 30 minutes, the reaction solution was discarded by pipetting, the cells were washed three times with PBST solution, and then the PBST solution was discarded by pipetting; 100 µL of TMB chromogenic solution was added to each well, and allowed to stand at 37°C for color development for 15 minutes;
(8) After 15 minutes, the chromogenic solution in the plate was discarded by pipetting to terminate the color development;
(9) A spot detection instrument ELISPOT (Model: Series 3B) was started, the cell culture plate after the color development reaction was placed on a sampling plate of the instrument, and the instrument was used to detect the number of blue cells in each well, that was, the number of infected cells; for detailed operating procedures of ELISPOT, the operating instructions of the instrument were referred.

H1-Hela cells that were not subjected to infection experiment were set simultaneously as the experimental control group.

Calculation method for rhinovirus titer: Infectious titer (IU/mL) = number of infected cells in detection well / amount of virus stock solution in detection well (mL), in which the number of infected cells in detection well = the average number of color-developed cells in detection wells - the average number of color-developed cells in negative wells. In the above formula, "number of infected cells in detection well" and the corresponding "amount of virus stock solution in detection well" were selected as the number of infected cells in detection well with the number of infected cells that was closest to 50 and not less than 50. The results were shown in Table 6, in which the amount of virus stock solution corresponded to the number of infected cells (an average of 3 wells), and the titer of virus could be calculated by substituting it into the above formula.

**Table 6: Relationship between infection amount of rhinovirus and number of infected cells in corresponding well**

| Amount of rhinovirus stock solution (10⁻³ mL) | 100 | 20 | 4 | 0.8 | 0.16 | 0.032 |
|---|---|---|---|---|---|---|
| Number of HRV-A2 infected cells | 1661 | 995 | 330 | 75 | 23 | 8 |
| Number of HRV-B 14 infected cells | 1681 | 939 | 229 | 69 | 24 | 9 |
| Number of HRV-C15 infected cells | 1656 | 816 | 303 | 63 | 39 | 6 |

According to the above results, it could be seen that the viral titers of three representative rhinoviruses, HRV-A2, HRV-B14 and HRV-C15, were 9.375 × 10⁴ (IU/mL), 8.625×10⁴ (IU/mL) and 7.875×10⁴ (IU/mL). The results of this example showed that the antisera induced by human rhinovirus universal affinity polypeptide 2C01 could be used to determine the infection titers of different representative rhinoviruses.

### Example 6: Preparation of antiserum against human rhinovirus

Preparation of immunogen: three 10 cm cell culture dishes were prepared and inoculated with H1-Hela cells. When the H1-Hela cells in the culture dish reached 80% confluence, the medium was changed into serum-free DMEM medium, and HRV-A2, HRV-B14 and HRV-C15 virus were inoculated, respectively; the inoculated virus dose was 10⁵ TCID₅₀, and the culture was carried out at 33°C after under slightly and evenly shaking. When the cells appeared completely diseased, the cells and supernatant were collected, and subjected to repeated freeze-thawing three times in a -80°C ultra-low temperature refrigerator. The cell debris were removed by centrifugation at 25,000 g for 10 minutes at 4°C; and the centrifuged virus supernatant was stored at -80°C for later use.

Basic immunity of mice: 6- to 8-week-old BALB/c female mice were injected with the above immunogen at multiple points, including subcutaneous injection on back, subcutaneous injection on groin, foot pad injection, intramuscular injection on limbs, etc. The injection dose was 500 µL/mouse/time. Five mice (numbered as Mouse 1 to Mouse 5) were immunized in parallel with each virus. Freund's complete adjuvant was mixed with the immunogen for the initial immunization, and then booster immunization was carried out every two weeks. Freund's incomplete adjuvant was mixed with the immunogen for the booster immunization. There were a total of two booster shots. On the 14^{th} day after the third shot of immunization, 200 µL of ocular venous blood was collected for titer determination.

### Example 7: Use of antiserum induced by polypeptide 2C01 to detect the titer of neutralizing antibody against human rhinovirus

In this example, the antiserum induced by affinity polypeptide 2C01 was used in the enzyme-linked immunospot method to detect the titer of neutralizing antibody against human rhinovirus. This method has the advantages of high efficiency and accuracy, which is more conducive to improving work efficiency and is more suitable for high-throughput detection of different subtypes of rhinovirus. The specific method was described as follows:
1. H1-Hela cells were plated in a 96-well cell culture plate (1×10⁴/well).
2. After 10 hours, the sample to be tested (monoclonal antibody sample, serum sample, ascites sample, cell culture supernatant sample, cell lysate sample, etc.) was diluted in gradient with serum-free DMEM (starting from the original solution, the dilution by 2 times was performed to obtain 8 gradients, and there were 3 repeat wells for each gradient).
3. 50 µL of the sample to be tested was taken from each well, mixed with 50 µL of rhinovirus (10⁴ TCID₅₀) diluted in serum-free DMEM and then incubated at 37°C for 2 h.
4. The culture medium in the 96-well cell culture plate pre-plated with H1-Hela cells was taken by pipetting, added with the incubated mixture, and cultured at 33°C for 14 to 24 hours, and then enzyme-linked immunospot reaction was performed.
5. The culture medium of each well was discarded by pipetting, then 100 µL of fixative solution (PBS solution containing 0.5% formaldehyde) was added to each well, and allowed to stand for 1 hour at room temperature in the dark.
6. The fixative solution was discarded by pipetting, then 100 µL of 1% Triton X-100 solution was added to each well to permeabilize the cells, and allowed to stand at room temperature for 30 minutes.
7. The permeabilization solution was discarded by pipetting, the cells were washed three times with PBST solution, and then the PBST solution was discarded by pipetting.
8. 100 µL of the antiserum induced by polypeptide 2C01 prepared in Example 2 was added to each well, in which the polypeptide-induced antiserum was diluted by 3000 times with enzyme diluent and allowed to stand at 37°C for reaction for 1 hour.
9. After 1 hour, the serum was discarded by pipetting, the cells were washed three times with PBST solution, and then the PBST solution was discarded by pipetting;
10. 100 µL of HRP-conjugated goat anti-mouse antibody diluted at 1:5000 was added to each well, and allowed to stand at 37°C for 30 minutes;
11. After 30 minutes, the reaction solution was discarded by pipetting, the cells were washed three times with PBST solution, and then the PBST solution was discarded by pipetting; 100 µL of TMB chromogenic solution was added to each well, and allowed to stand at 37°C for color development for 15 minutes.
10. After 15 minutes, the chromogenic solution in the plate was discarded by pipetting to terminate the color development.
11. A spot detection instrument ELISPOT was started to detect the number of blue cells in each well, which was the number of infected cells. For detailed operating procedures, the operating instructions of the ELISPOT instrument were followed.

At the same time, the H1-Hela cells that were not infected in the experiment were set as negative wells, the H1-Hela cells that were only infected with the virus were set as positive wells, and there were 5 wells set on each 96-well plate.

The maximum dilution factor that achieved an infection inhibition rate up to 50% was used as the neutralizing titer of the sample. The infection inhibition rate of each sample = (1 - the number of infected cells in the sample well/(the total number of colored cells in positive wells/5 - the total number of colored cells in negative wells/5)) × 100%. The number of infected cells in the sample well = the average number of colored cells in the sample well - the total number of colored cells in the negative wells/3.

In this example, 6 samples of polyantiserum from immunized mouse prepared in Example 6 were randomly selected for the detection of neutralizing antibody against human rhinovirus. The neutralizing antibody detection results (shown in Table 7) showed that the antisera induced by polypeptide 2C01 could be used to detect the titer of neutralizing antibody against human rhinovirus; the polyantiserum from immunized mouse could neutralize HRV-A2, HRV-B14 or HRV-C15 virus.

**Table 7. Detection results of neutralizing antibody titer of polyantiserum from the immunized mouse**

| Immunogen | Mouse No. | neutralizing antibody titer against HRV-A2 | neutralizing antibody titer against HRV-B 14 | neutralizing antibody titer against HRV-C15 |
|---|---|---|---|---|
| HRV-A2 virus | Mouse 1 | 512 | 0 | 0 |
| HRV-A2 virus | Mouse 2 | 1024 | 0 | 0 |
| HRV-B14 virus | Mouse 1 | 0 | 1024 | 0 |
| HRV-B14 virus | Mouse 2 | 0 | 256 | 0 |
| HRV-C15 virus | Mouse 1 | 0 | 0 | 128 |
| HRV-C15 virus | Mouse 2 | 0 | 0 | 1024 |

### Example 8: Fusion expression of human rhinovirus universal affinity epitope polypeptide and HBcAg protein

### 1. Construction of pTO-T7-C149 expression vector

It is well known by those skilled in the art that HBcAg has strong T cell immunogenicity, and the fusion of exogenous peptide in MIR (mayor immunodominant region, aa 78-83) inside HBcAg would not change the polymerization properties, antigenicity and immunogenicity of its particles, and could expose foreign epitopes to the particle surface (see, Fu et al., (2009). Comparative immunogenicity evaluations of influenza A virus M2 peptide as recombinant virus like particle or conjugate vaccines in mice and monkeys. Vaccine 27 (9): 1440-1447; Jin et al., (2007). Protective immune responses against foot-and-mouth disease virus by vaccination with a DNA vaccine expressing virus-like particles. Viral Immunol 20 (3): 429-440; and Yin et al., (2010). Hepatitis B virus core protein as an epitope vaccine carrier: a review. Sheng Wu Gong Cheng Xue Bao 26 (4): 431-438.).

Taking advantage of the property that 1-149 aa of HBcAg could be expressed in the form of virus-like particles in *E. coli,* the inventors inserted the 149 aa into the *E. coli* expression vector pTO-T7 (for the preparation steps of this vector, see Luo Wenxin and Zhang Jun, Yang Haijie et al., Construction and application of an Escherichia coli high effective expression vector with an enhancer [J]. Chinese Journal of Biotechnology, 2000, 16 (5): 578-581) (other commonly used *E*. *coli* expression vectors in this field could also be used), and the 79^{th} and 80^{th} amino acids (the corresponding amino acids were PA) of the B cell dominant epitope segment in HBcAg were replaced with a linker (SEQ ID NO: 8), and GGATCC was introduced at 265 nt to 270 nt, GAATTC was introduced at 274 nt to 279 nt (relative to pTO-T7-C149) so as to introduce two restriction endonuclease recognition sites (BamHI and EcoRI) to construct the mutant HBc expression plasmid pTO-T7-C149. The exogenous protein can be inserted at the 93^{rd} amino acid position of C149.

Primers were designed for the human rhinovirus (W10 (HRV-C15)) non-structural protein segments 2C (aa 151-163) (2C01, SEQ ID NO:1), 2C (aa 12-20) (2C02, SEQ ID NO:2) and 2C (aa 186-193) (2C03, SEQ ID NO:3) (the primer sequences were shown in Table 8), and the peptides 2C01, 2C02 and 2C03 were inserted into the HBcAg protein respectively to obtain fusion proteins called C149-2C (aa 151-163) or C149-2C01 (SEQ ID NO: 5), C149-2C (aa 12-20) or C149-2C02 (SEQ ID NO: 6), C149-2C (aa 186-193) or C149-2C03 (SEQ ID NO: 7). Among them, the amino acid sequence of human rhinovirus (W10 (HRV-C15)) non-structural protein 2C was shown in SEQ ID NO: 9.

**Table 8: Primer sequences used for fusion proteins cloning**

| Protein name | Embedded fragment | Primer sequence (5' to 3') | SEQ ID NO: |
|---|---|---|---|
| C149-2C (aa 151-163) (C149-2C01) | 2C (aa 151-163) (2C01) | GATCCTACTCACTACCACCTGATCC CAAATATTTCGATGGTTATG | 10 |
| | | AATTCATAACCATCGAAATATTTGG GATCAGGTGGTAGTGAGTAG | 11 |
| C149-2C (aa 12-20) (C149-2C02) | 2C (aa 12-20) (2C02) | GATCCTGCAATGCAGCAAAAGGACT TGAATGGG | 12 |
| | | AATTCCCATTCAAGTCCTTTTGCTGC ATTGCAG | 13 |
| C149-2C (aa 186-193) (C149-2C03) | 2C (aa 186-193) (2C03) | GATCCTTCTGTCAGATGGTATCAAC AACAG | 14 |
| | | AATTCTGTTGTTGATACCATCTGACA GAAG | 15 |

By overlapping the upstream and downstream primers of the synthesized 2C segment, a fragment with BamHI and EcoR I sticky ends was obtained (for the specific method steps of cloning construction, see, Xu L, et al. 2014. Protection against Lethal Enterovirus 71 Challenge in Mice by a Recombinant Vaccine Candidate Containing a Broadly Cross-Neutralizing Epitope within the VP2 EF Loop. Theranostics 4:498-513.). 1 µL of each upstream and downstream primers was taken, added with 48 µL of ddH₂O at the same time, mixed and reacted at 94°C for 4 minutes, then allowed to stand at 70°C for reaction for 10 minutes, and slowly cooled to room temperature. At the same time, the vector pTO-T7-C149 was double-digested with BamHI and EcoR I, and the vector was recovered and ligated with the overlapped fragments. The ligation product was transformed into *E.coli* ER2566 (purchased from NEB), and subjected to expression identification and plasmid digestion identification (at the same time, pTO-T7-C149 was used as a blank control). The plasmids correctly identified were inserted with the required target fragments, and named as C149-2C (aa 151-163) or C149-2C01 (SEQ ID NO: 5), C149-2C (aa 12-20) or C149-2C02 (SEQ ID NO: 6), C149-2C (aa 186-193) or C149-2C03 (SEQ ID NO: 7).

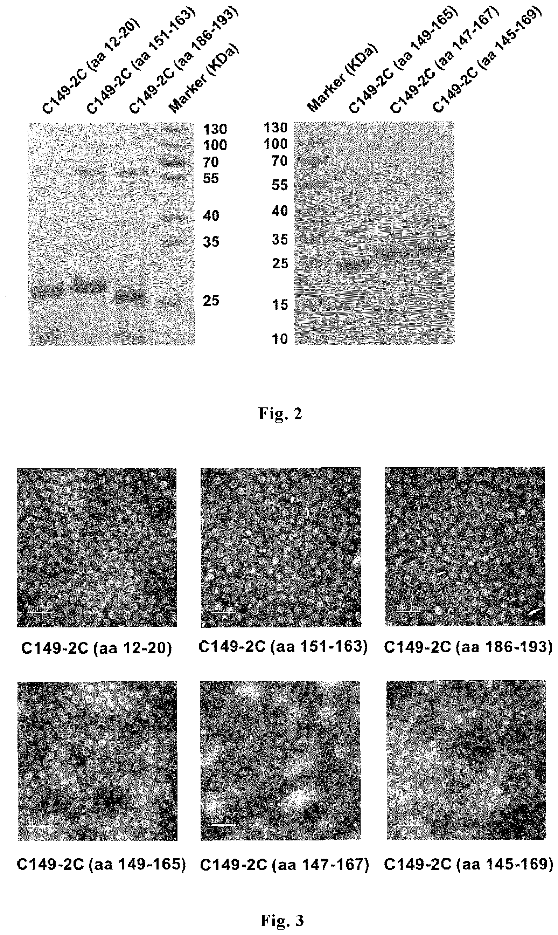

The ER2566 strain containing the above expression plasmid and the ER2566 strain containing the pTO-T7-C149 empty plasmid were cultured under shaking at 37°C until the OD600 was about 0.5, and then transferred and expanded to 500 mL of LB (containing kanamycin sulfate) at a ratio of 1:1000, cultured until OD600 was about 0.8, added with 500 µL of IPTG, and induced at 30°C for 6 h. The bacterial cells were collected at 4°C and centrifuged at 8000 rpm for 10 minutes. The supernatant was discarded, and the bacterial pellets were resuspended in TB 8.0 buffer, 10 mL/bottle. Under ice-water bath, the cells were disrupted by an ultrasonic disrupter under ultrasonic conditions: working time: 3 min/bottle; pulse: 2 sec on, 4 sec off; output power: 55%. After centrifugation at 12,000 rpm for 10 min, the supernatant was retained. The identification by SDS-PAGE showed that all fusion proteins were expressed in the supernatant (SDS-PAGE results were shown in Fig. 2).

Since the expression supernatant contained many impurities, the inventors used the following method to perform the purification and promote the spontaneous assembly of proteins to form particles: the supernatant obtained after ultrasonic treatment and centrifugation was thermally denatured in a 65°C water bath for 30 minutes, centrifuged at 12000 rpm for 10 minutes, and the supernatant was collected. The supernatant and saturated ammonium sulfate solution were mixed in equal volumes and allowed to stand in an ice-water bath for 30 min, then centrifuged at 12,000 rpm for 10 minutes, and the precipitate was retained. The purified protein was then promoted to spontaneously assemble into particles in PBS buffer, and then observed under an electron microscope. The successfully assembled particles were in the form of uniform hollow spheres. The electron microscope observation results were shown in Fig. 3.

The purified and assembled fusion protein was used to immunize 6-week-old BALB/c female mice. Each displayed peptide was used to immunize 5 mice in parallel (numbered as: Mouse 1 to Mouse 5). The immunization dose was 100 µg/mouse. Freund's complete adjuvant was mixed with the assembled fusion protein for the initial immunization, and booster immunization was performed every two weeks thereafter. Freund's incomplete adjuvant was mixed with the assembled fusion protein for the booster immunization, and there were a total of three booster shots. Blood samples were collected on the 14^{th} day after the third booster shot.

### Example 9: Binding activity of immune sera induced by fusion proteins C149-2C01, C149-2C02, C149-2C03 to human rhinovirus

The antisera induced by fusion protein C149-2C01, C149-2C02, C149-2C03 obtained in Example 8 were used to detect different representative rhinoviruses using immunofluorescence. The steps of the immunofluorescence method were the same as those in Example 4, and the experimental results were shown in Table 9.

**Table 9: Binding activity of immune sera induced by C149-2C01, C149-2C02, C149-2C03 to H1-Hela cells infected with rhinovirus, as detected by immunofluorescence**

| | C149-2C01 | C149-2C02 | C149-2C03 | PBS |
|---|---|---|---|---|
| HRV-A1B | + | - | - | - |
| HRV-A2 | + | - | - | - |
| HRV-A9 | + | - | - | - |
| HRV-A16 | + | - | - | - |
| HRV-A21 | + | - | - | - |
| HRV-A23 | + | - | - | - |
| HRV-A29 | + | - | - | - |
| HRV-A34 | + | - | - | - |
| HRV-A36 | + | - | - | - |
| HRV-A40 | + | - | - | - |
| HRV-A43 | + | - | - | - |
| HRV-A49 | + | - | - | - |
| HRV-A54 | + | - | - | - |
| HRV-A59 | + | - | - | - |
| HRV-A77 | + | - | - | - |
| HRV-A78 | + | - | - | - |
| HRV-A89 | + | - | - | - |
| HRV-B5 | + | - | - | - |
| HRV-B6 | + | - | - | - |
| HRV-B14 | + | - | - | - |
| HRV-B17 | + | - | - | - |
| HRV-C11 | + | - | - | - |
| HRV-C15 | + | + | + | - |
| HRV-C23 | + | - | - | - |

| | | | | |
|---|---|---|---|---|
| Note: +, positive; -, negative | | | | |

The results proved that the antiserum induced by C149-2C01 fusion protein polypeptide could cross-bind to 24 representative rhinoviruses (HRV-A1B, HRV-A2, HRV-A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV-C15 and HRV-C23); the infected H1-Hela cells showed green fluorescence, while the control of H1-Hela cells not infected with rhinovirus showed no response; while the antisera induced by C149-2C02 and C149- 2C03 fusion protein could not specifically cross-bind to different subtypes of human rhinovirus. This result showed that the carrier protein could present the epitope polypeptide of the present invention in the form of virus-like particles. Based on the above experimental results, the 2C01 polypeptide and its fusion protein C149-2C01 were selected for the next experiment.

### Example 10: Fusion expression of HBcAg protein and variants of human rhinovirus universal affinity epitope polypeptide with extended length

According to the method described in Example 8, primers were designed for the variants of human rhinovirus (W10 (HRV-C15)) non-structural protein 2C01 with extended lengths: 2C (aa149-165) (2C09, SEQ ID NO: 35), 2C (2C10, aa147-167) (SEQ ID NO: 36) and 2C (2C11, aa145-169) (SEQ ID NO: 37) segments (the primer sequences were shown in Table 10), and the variants were inserted into the HBcAg protein respectively to obtain fusion proteins that were respectively called C149-2C (aa149-165) or C149-2C09 (SEQ ID NO: 49); C149-2C (aa147-167) or C149-2C10 (SEQ ID NO: 50); C149-2C (aa145-169) or C149-2C11 (SEQ ID NO: 51).

In addition, the fusion proteins C149-2C04 (SEQ ID NO: 44), C149-2C05 (SEQ ID NO: 45), C149-2C06 (SEQ ID NO: 46), C149-2C07 (SEQ ID NO: 47), C149 -2C08 (SEQ ID NO: 48) inserted with polypeptides 2C04 (SEQ ID NO: 30), 2C05 (SEQ ID NO: 31), 2C06 (SEQ ID NO: 32), 2C07 (SEQ ID NO: 33) or 2C08 (SEQ ID NO: 34) were obtained by the same method.

**Table 10: Primer sequences used for the cloning of fusion proteins comprising the variants with extended length**

| Protein name | Embedde d fragment | Primer sequence (5' to 3') | SEQ ID NO: |
|---|---|---|---|
| C149-2C (aa 149-165) | 2C (aa 149-165) | GATCCCAGGTATACTCACTACCACCTgATCCCAAA TATTTCGATGGTTATGACCAAG | 38 |
| | | AATTCTTGGTCATAACCATCGAAATATTTGGGATC AGGTGGTAGTGAGTATACCTGG | 39 |
| C149-2C (aa 147-167) | 2C (aa 147-167) | GATCCGAGGCACAGGTATACTCACTACCACCTGAT CCCAAATATTTCGATGGTTATGACCAACAGCAGG | 40 |
| | | AATTCCTGCTGTTGGTCATAACCATCGAAATATTTG GGATCAGGTGGTAGTGAGTATACCTGTGCCTCG | 41 |
| C149-2C (aa 145-169) | 2C (aa 145-169) | GATCCACCCAGGAGGCACAGGTATACTCACTACC ACCTGATCCCAAATATTTCGATGGTTATGACCAAC AGCAGGTTGTCGG | 42 |
| | | AATTCGACAACCTGCTGTTGGTCATAACCATCGAA ATATTTGGGATCAGGTGGTAGTGAGTATACCTGTG CCTCCTGGGTG | 43 |

By overlapping the synthesized upstream and downstream primers for the segment of variant of 2C with extended length, the expression and identification of C149-2C fusion protein comprising the variant with extended length was performed according to the fusion protein expression method in Example 8. The finally obtained fusion protein expression supernatant was subjected to SDS-PAGE identification, indicating that all extended variant fusion proteins were expressed in the supernatant (SDS-PAGE results were shown in Fig. 2).

The obtained fusion protein was purified using the method described in Example 8, and then the purified protein was promoted to spontaneously assemble into particles in PBS buffer, and then observed under an electron microscope. The successfully assembled particles were in the form of uniform hollow spheres. The results were shown in Fig. 3.

The purified and assembled fusion protein was used to immunize 6-week-old BALB/c female mice. Each displayed peptide was used to immunize 5 mice in parallel (numbered as: Mouse 1 to Mouse 5). The immunization dose was 100 µg/mouse. Freund's complete adjuvant was mixed with the assembled fusion protein for the initial immunization, and booster immunization was performed every two weeks thereafter. Freund's incomplete adjuvant was mixed with the assembled fusion protein for the booster immunization, and there were a total of three booster shots. Blood samples were collected on the 14^{th} day after the third booster shot.

### Example 11: Binding activity of immune sera induced by fusion proteins C149-2C (aa 149-165), C149-2C (aa 147-167), C149-2C (aa 145-169) to human rhinovirus

The obtained antisera induced by fusion proteins C149-2C (aa 149-165), C149-2C (aa 147-167), and C149-2C (aa 145-169) were used to detect different representative rhinoviruses by immunofluorescence. The steps of the immunofluorescence method were as in Example 4, and the experimental results were shown in Table 11.

**Table 11: Binding activity of immune sera induced by C149-2C (aa 149-165), C149-2C (aa 147-167), C149-2C (aa 145-169) to rhinovirus-infected H1-Hela cells, as detected by immunofluorescence**

| | C149-2C (aa 149-165) | C149-2C (aa 147-167) | C149-2C (aa 145-169) | PBS |
|---|---|---|---|---|
| HRV-A1B | + | + | + | - |
| HRV-A2 | + | + | + | - |
| HRV-A9 | + | + | + | - |
| HRV-A16 | + | + | + | - |
| HRV-A21 | + | + | + | - |
| HRV-A23 | + | + | + | - |
| HRV-A29 | + | + | + | - |
| HRV-A34 | + | + | + | - |
| HRV-A36 | + | + | + | - |
| HRV-A40 | + | + | + | - |
| HRV-A43 | + | + | + | - |
| HRV-A49 | + | + | + | - |
| HRV-A54 | + | + | + | - |
| HRV-A59 | + | + | + | - |
| HRV-A77 | + | + | + | - |
| HRV-A78 | + | + | + | - |
| HRV-A89 | + | + | + | - |
| HRV-B5 | + | + | + | - |
| HRV-B6 | + | + | + | - |
| HRV-B14 | + | + | + | - |
| HRV-B17 | + | + | + | - |
| HRV-C11 | + | + | + | - |
| HRV-C15 | + | + | + | - |
| HRV-C23 | + | + | + | - |

| | | | | |
|---|---|---|---|---|
| Note: +, positive; -, negative | | | | |

The results proved that the antisera induced by C149-2C (aa 149-165), C149-2C (aa 147-167), and C149-2C (aa 145-169) fusion protein polypeptide were able to cross-bind to 24 representative rhinoviruses (HRV-A1B, HRV-A2, and HRV- A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV-C15, and HRV-C23); the infected H1-Hela cells showed green fluorescence, while the H1-Hela cell control that was not infected with rhinovirus showed no response. This result showed that not only the C149-2C01 carrier protein could present the epitope polypeptide of the present invention in the form of virus-like particles, but also the extended segments C149-2C (aa 149-165), C149- 2C (aa 147-167) and C149-2C (aa 145-169) along original segments thereof could present the variant epitope polypeptide with extended length of the present invention in the form of virus-like particles.

### Example 12: Use of immune serum induced by fusion protein C149-2C01 to detect infection and titer of human rhinovirus

The antiserum induced by C149-2C01 fusion protein obtained in Example 8 was used to detect the titer of rhinoviruses (HRV-A2, HRV-B14 and HRV-C15) by the gradient dilution spot counting method. The specific steps were as in Example 5, and the experimental results were shown in Table 12 below.

**Table 12: Relationship between infection dose of rhinovirus and number of infected cells in corresponding wells**

| Amount of rhinovirus stock solution (10⁻³ mL) | 100 | 20 | 4 | 0.8 | 0.16 | 0.032 |
|---|---|---|---|---|---|---|
| Number of HRV-A2 infected cells | 1764 | 925 | 295 | 45 | 15 | 6 |
| Number of HRV-B14 infected cells | 1584 | 839 | 322 | 71 | 26 | 7 |
| Number of HRV-C15 infected cells | 1921 | 1016 | 424 | 123 | 49 | 7 |

According to the above results, it could be seen that the viral titers of three representative rhinoviruses, HRV-A2, HRV-B14 and HRV-C15, were 7.375×10⁴ (IU/mL), 8.875×10⁴ (IU/mL) and 1.538×10⁵ (IU/mL), respectively. The results of this example showed that the antiserum induced by the fusion protein C149-2C01 could be used to determine the infection titers of different representative rhinoviruses.

### Example 13: Use of immune serum induced by fusion protein C149-2C01 to detect the titer of neutralizing antibody against human rhinovirus

By using the enzyme-linked immunospot method, in this example, the antiserum induced by C149-2C01 fusion protein obtained in Example 8 was used to detect the titer of neutralizing antibody against human rhinovirus. The specific methods were as in Example 7.

In this example, 6 samples of polyantiserum from the immunized mouse prepared in Example 6 were randomly selected for the detection of neutralizing antibody against human rhinovirus. The neutralizing antibody detection results were shown in Table 13. The results showed that immune serum induced by C149-2C01 could be used to detect the titer of neutralizing antibody against human rhinovirus; the polyantiserum from the immunized mouse could neutralize HRV-A2, HRV-B14 or HRV-C15.

**Table 13: Detection results of neutralizing antibody titer of polyantiserum from the immunized mouse**

| Immunogen | Mouse No. | neutralizing antibody titer against HRV-A2 | neutralizing antibody titer against HRV-B14 | neutralizing antibody titer against HRV-C15 |
|---|---|---|---|---|
| HRV-A2 virus | Mouse 1 | 512 | 0 | 0 |
| HRV-A2 virus | Mouse 2 | 1024 | 0 | 0 |
| HRV-B14 virus | Mouse 1 | 0 | 1024 | 0 |
| HRV-B14 virus | Mouse 2 | 0 | 256 | 0 |
| HRV-C15 virus | Mouse 1 | 0 | 0 | 128 |
| HRV-C15 virus | Mouse 2 | 0 | 0 | 1024 |

### Example 14: Preparation of monoclonal antibody against human rhinovirus universal affinity epitope polypeptide

(1) Mouse immunization
   1. Preparation of immunogen: The fusion protein C149-2C01 prepared in Example 8 was used as the immunogen, Freund's complete adjuvant was used for the initial immunization, Freund's incomplete adjuvant was used for the subsequent booster immunization, and no adjuvant was added for the last booster 72 hours before the fusion.
   2. Basic immunity of mice: The immunogen prepared in step 1 above was injected into 6- to 8-week-old BALB/c female mice at multiple points, including subcutaneous injection on the back, subcutaneous injection in the groin, foot pad injection, intramuscular injection in the limbs, etc. The injection dose was 500 µL/animal/time. Booster immunization was performed every 2 weeks. Before each immunization, 100 to 200 µL of orbital venous blood was collected for titer determination. When the mouse serum titer reached the plateau stage, the immunization was stopped, and the cell fusion experiment was performed after two months of rest.
   3. Boosting immunity 72 hours before cell fusion was very important to stimulate antibody response, and mice needed to be boosted 72 hours before fusion. The mice were directly spleen immunized with 100 µL of 0.5 mg/mL recombinant protein (C149-2C01). Before spleen immunization, the mice needed to be anesthetized with ether, the abdominal skin was cut open and the spleen was taken out, 100 µL of antigen was injected longitudinally along the spleen, and the abdominal skin incision was quickly sutured.
(2) Preparation and screening of fusion hybridoma cells
   1. Preparation of mouse macrophages: (i) A BALB/c mouse about 6-week-old was killed, rinsed with tap water, and soaked in 75% ethanol solution for 5 minutes. The mouse was placed on a clean workbench with its belly facing up. A tweezer was used to lift the mouse abdominal skin and a small cut was made. Then two large tweezers were used to tear the skin upward and downward to fully expose the abdomen. (ii) The peritoneum was lifted with a sterile forcep, a pair of scissors was used to cut a small opening in the center of the peritoneum, a 1 mL pipette was used to inject an appropriate amount of culture solution into the abdominal cavity through the small opening, a pipette was used to carefully stir the peritoneal cavity, and finally the culture solution was pipetted out and placed in a centrifuge tube. (iii) The peritoneal cell fluid was dissolved in HAT culture medium or HT culture medium to a final concentration of 2×10⁵/mL. (iv) The cells were added to a 96-well cell culture plate and cultured in an incubator, or were directly mixed with the fused cells and then added to a 96-well cell culture plate.
   2. Preparation of mouse thymocytes: (i) A BALB/c mouse about 13 days old was sacrificed by neck breaking, rinsed with tap water, and soaked in 75% ethanol solution for 5 minutes. The mouse was placed on a clean workbench with its belly facing up. (ii) A tweezer was used to lift the abdominal skin of the mouse and the outer skin of the abdomen and chest was cut. (iii) Another pair of clean scissors was used to cut open the chest cavity and a tweezer was used to remove the milky white thymus. The thymus was ground and passed through a 200-mesh cell sieve to obtain thymus feeder cell fluid.
   3. Preparation of mouse myeloma cells: (i) The mouse myeloma cell line Sp2/0-Ag14 (Sp2/0) is easy to culture and has a high fusion rate. It is currently the most ideal fusion cell. However, it should be noted that the cell line would poorly grow under conditions of excessive dilution (density below 3×10⁵/mL) and alkaline pH (pH>7.3). (ii) Myeloma cells (purchased from ATCC) were cultured with 20% FBS RPMI-1640 culture medium. When the number of cells was 10⁴ to 10⁶/mL, the cells grew logarithmically. At this time, the cells were round and translucent, uniform in size, clear in edge, neatly arranged, and semi-densely distributed. Generally, when cells were in the mid-logarithmic growth phase (about 1 to 5×10⁵/mL), they could be diluted and passaged at a ratio of 1:5 to 1:10. The cells in the logarithmic growth phase with vigorous growth and good shape were selected for fusion. The viable cell number of fused myeloma cells should reach more than 95%. (iii) Before fusion, the myeloma cells were moved from the culture bottle into a centrifuge tube and washed three times with RPMI-1640 culture medium (1000 rpm, 5 min). The cells were resuspended in RPMI-1640 culture medium and counted. (iv) Generally, the mouse myeloma cells were recovered from 5 days before fusion. Each fusion required about 6 bottles of 35 cm² Sp2/0 cells.
   4. Preparation of immune spleen cells: (i) The BALB/c mouse to be fused as prepared in the above step (1) was taken, the eyeballs were removed to bleed it to death, the blood was collected to make antiserum, which could be used as a positive control for antibody detection. The mouse was rinsed with tap water and soaked in 75% ethanol solution for 5 minutes, and then placed in right-side decubitus position on a dissecting board in a super clean bench. (ii) The abdominal cavity was opened and the spleen was taken out. A pair of scissors was used to cut it into small pieces which were then placed on a 200-mesh cell screen, and then squeezed and ground with a grinding rod (syringe inner core). RPMI-1640 culture medium was added dropwise with a pipette. (iii) An appropriate amount of RPM-1640 culture medium was supplemented, allowed to stand for 3 to 5 minutes, and 2/3 of the suspension was transferred into a 50 mL plastic centrifuge tube. The above process was repeated 2 to 3 times. (iv) The cells were washed three times with RPMI-1640 culture medium (centrifuged at 1000 rpm for 10 min). (v) The cells were resuspended in RPMI-1640 culture medium and counted.
   5. Preparation of hybridoma cells by fusion with PEG fusogen: (i) Before fusion, 1 mL of PEG-1500, 10 mL of RPMI-1640 serum-free medium and 200 mL of complete medium were balanced to 37°C. (ii) The prepared myeloma cells and spleen cells were mixed in a 50 mL centrifuge tube (1×10⁸ spleen cells and 1×10⁷ myeloma cells, about 10:1), centrifuged at 1500 rpm for 8 minutes, and the tube was gently tapped at bottom to loosen the cells into a paste. (iii) 0.8 mL of PEG was added into the centrifuge tube, the addition was completed within 60 s under gent agitation while adding. Then, 10 mL of RPM-1640 complete culture medium pre-warmed to 37°C was added and stirred gently. Finally, RPMI-1640 culture medium was added to 40 mL, and centrifuged at 1000 rpm for 5 minutes. (iv) The supernatant was discarded, a small amount of HT culture medium was taken to disperse the cells, and then added with the prepared HT culture medium. The cells were added to a 96-well cell culture plate, 100 µL per well, and cultured in a CO₂ incubator. (v) After 12 hours, HAT complete medium was prepared and added dropwise to wells, 100 µL per well. After 5 days, HT complete medium was used to replace 50% to 100% of the cell supernatant in the wells. After 9 to 14 days, the supernatant was pipetted for detection.
   6. Screening of hybridomas: Indirect ELISA screening was adopted, coating 100 ng/well of recombinant antigen was performed, 50 µL of cell supernatant was added for detection, and positive clone wells were selected.
   7. Cloning of hybridoma cells: There were usually two or more hybridoma colonies in the selected culture wells containing the positive supernatant. Some of the colonies might not secrete antibody or might secrete antibody not required for the experiment. Only one of the colonies was of hybridoma cells secreting specific antibody. Therefore, they must be separated by clonal culture method to select the required hybridoma cells. The most commonly used clonal culture method was the limiting dilution method. The cells were first diluted in gradient at a certain concentration, and then inoculated into a 96-well cell culture plate, so that only one cell grew in the well as much as possible. Generally, the hybridoma monoclonal positive cell strain needed to be cloned 2 to 3 times until they were 100% positive, which was considered a stable clone. The stable hybridoma cell strain 9A5 was obtained, its deposit number was CCTCC No. C2021141, its deposit date was May 28, 2021, it was deposited in the China Center for Type Culture Collection (CCTCC), and the deposit address is Luojia, Wuchang, Wuhan City Mountain (post code: 430072).
(3) Induction of ascites with monoclonal antibody
   1. 2 to 3 BALB/c mice were taken, and 0.5 mL of liquid paraffin oil was injected into the abdominal cavity.
   2. After 1 week, the hybridoma cells in the logarithmic growth phase were treated, centrifuged at 1000 rpm for 5 minutes, and the supernatant was discarded. The hybridoma cells were suspended in serum-free culture medium, and the cell number was adjusted to (1 to 2) × 10⁶/mL. Each mouse was injected intraperitoneally with 0.5 mL.
   3. After 7 to 10 days, when the abdomen of the mouse became obviously enlarged, the mouse was sacrificed by neck breaking. The mice were rinsed with tap water and immersed in 75% ethanol for 5 minutes. With the mouse's abdomen facing up, four injection needles were used to fix the mouse's limbs on the dissecting table. A tweezers was used to lift the skin of the mouse's abdomen and a small cut was made. Then the cut was made from both sides toward the back of the mouse, and two large tweezers were used to tear open the skin to fully expose the abdomen. The peritoneum was lifted with a sterile ophthalmic forcep, a small opening was cut in the center of the peritoneum, and then a 1 mL suction pipette was used to suck out all the ascites in the abdominal cavity through the small opening. All the ascites was placed in a centrifuge tube, centrifuged at 3000 rpm for 20 min, and then the supernatant was collected.
(4) Purification of monoclonal antibody ascites: The ascites was precipitated with octanoic acid-saturated ammonium sulfate and purified by Protein A affinity chromatography (purchased from GE Company in the United States) to obtain purified monoclonal antibody 9A5.
(5) Identification of antibody subtypes: The fusion protein antigen was diluted 100 times with 20 mM PB 7.4, plated on a 96-well enzyme plate, 100 µL/well, at 37°C for 2 h, the plate was washed once with PBST, and non-specific binding sites were blocked with relevant blocking solution (20 mM PB 7.4 containing 150 mM NaCl, 0.5% casein, 0.002% gelatin), 200 µL/well, at 4°C overnight. 100 µL of monoclonal antibody diluted 500 times was taken and added to an enzyme plate, and incubated at 37°C for 1 h. The plate was washed 5 times with PBST, and added with HRP-labeled anti-IgG1, IgG2a, IgG2b, IgG3 and IgM goat anti-mouse secondary antibodies, respectively, and incubated at 37°C for 30 minutes. The plate was washed 5 times with PBST, added with TMB chromogenic solution for color development for 15 minutes, a stop solution was used for termination, and a microplate reader (TECAN sunrise) was used for reading values. The results showed that monoclonal antibody 9A5 was of IgG2a subtype.
(6) HRP labeling of monoclonal antibody: All operations were performed under light-proof conditions: (i) 2 mg of HRP dry powder was weighed and dissolved in 0.1 mL of ddH₂O. (ii) 2 mg of 9A5 antibody was weighed and dissolved in 0.1 mL of ddH₂O. 0.1 mL of NaIO₄ solution was taken and mixed slowly with 0.1 mL of HRP, and allowed to stand at 4°C for 30 minutes. (iii) 2 µL of ethylene glycol was added, and allowed to stand in the dark at room temperature for 30 minutes. (iv) An equal volume of (0.2+0.2+0.05 mL) of CB buffer (pH 9.6, 50 mM) was added to make the pH of the solution under alkaline conditions. (v) The reaction solution was moved from the centrifuge tube to a dialysis bag containing the antibody, dialyzed with CB buffer at 4°C for 4 hours, and the medium was changed once. (vi) 0.4 mg of NaBH₄ was dissolved in 20 µL of ddH₂O and transferred into the dialysis bag, allowed to stand at 4°C for 2 hours, and shaken every 30 minutes. (vii) An equal volume of 50% saturated ammonium sulfate (SAS) solution was added, allowed to precipitate at 4°C for 1 hour, centrifuged at 12,000 rpm for 10 minutes, and the supernatant was discarded (the SAS should be carefully and clearly removed by inversion to prevent the residual SAS from affecting the antibody). Finally, 500 µL of buffer (50% glycerol and 10% NBS) was added to resuspend the pellet, and stored at -20°C.
(7) Characteristic analysis of anti-C149-2C01 mouse monoclonal antibody (9A5)
   1. The synthesized polypeptides (2C01, 2C02, 2C03) of Example 2 were plated to a 96-well ELISA plate at a concentration of 1 µg/well, respectively. After the plate was washed once, a blocking solution (20 mM PB7.4 containing 150 mM NaCl, 0.5% casein, 0.002% gelatin) was used to block non-specific binding sites, the blocking was performed at 37°C for 2 hours, then it was spin-dried for later use.
   2. The antibody 9A5 was diluted to a concentration of 2 µg/mL (the diluent had the same formula as the blocking solution), 100 µL thereof was added to each well, and incubated at 37°C for 30 minutes. The plate was washed 5 times with PBST, added with HRP-conjugated goat anti-mouse antibody diluted at 1:5000, and allowed to stand at 37°C for reaction for 30 minutes. The plate was washed 5 times with PBST, added with a chromogenic solution to perform color development for 15 minutes, added with a stop solution for termination, and placed on a microplate reader for reading values.
   3. The fusion proteins (C149-2C01, C149-2C02, C149-2C03) prepared in Example 8 were plated to a 96-well ELISA plate at a concentration of 200 ng/well, respectively. After the plate was washed once, a blocking solution was used to block non-specific binding sites, the blocking was performed at 37°C for 2 hours, and then it was spin-dried for later use.
   4. After the enzyme-labeled antibody obtained in step (6) above was diluted at 1:1000 (the diluent formula was the same as the blocking solution), 100 µL thereof was added to each well, and incubated at 37°C for 30 minutes. The plate was washed 5 times with PBST, added with a chromogenic solution for color development for 15 minutes, added with a stop solution for termination, and placed on a microplate reader for read values.

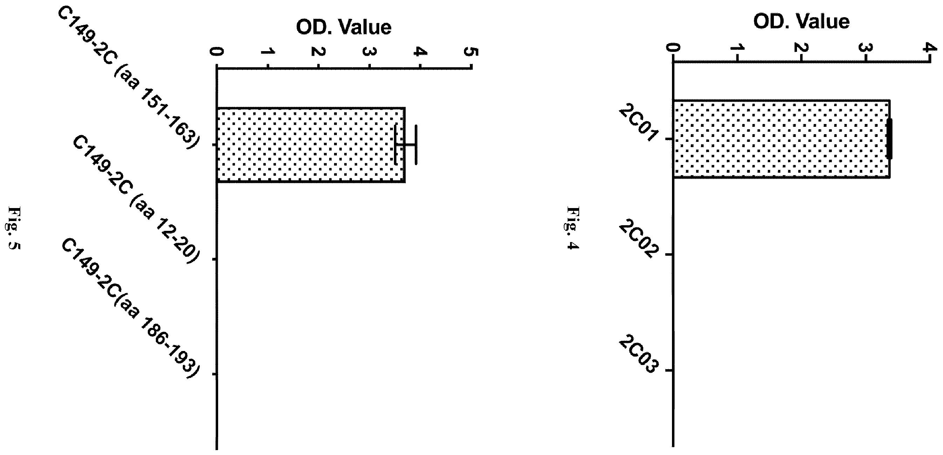

The results were shown in Figs. 4 and 5. The results in Fig. 4 showed that the 9A5 antibody could bind to the 2C01 polypeptide and had good activity. The results in Fig. 5 showed that the 9A5-HRP enzyme-labeled antibody could bind to the C149-2C01 recombinant protein and had good activity. The following examples were completed using 9A5 or 9A5-HRP.

### Example 15: Binding activity of monoclonal antibody 9A5 to human rhinovirus

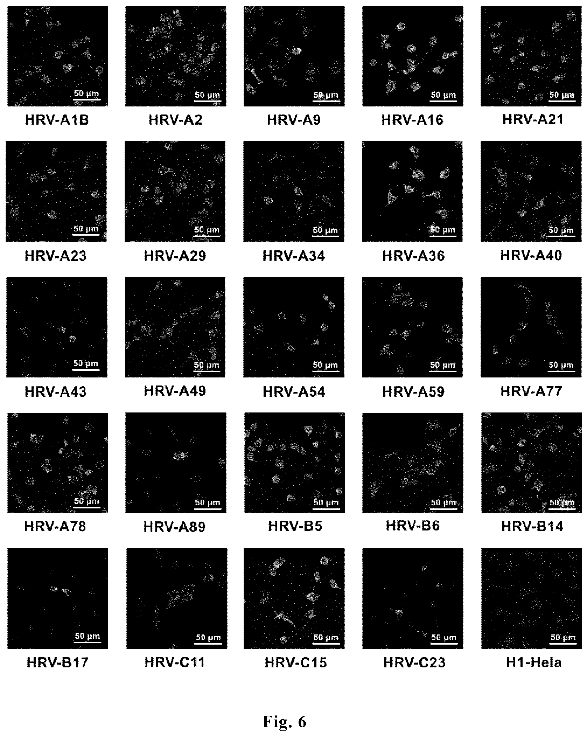

The monoclonal antibody 9A5 prepared in Example 14 was used to detect different representative rhinoviruses using immunofluorescence method. The steps of the immunofluorescence method were as in Example 4, and the experimental results were shown in Fig. 6. The results showed that 9A5 could cross-bind to H1-Hela cells infected with 24 rhinoviruses (HRV-A1B, HRV-A2, HRV-A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV-C15 and HRV-C23), and showed green fluorescence, while the control of H1-Hela cells not infected with rhinovirus showed no response, indicating that monoclonal antibody 9A5 could specifically cross-bind to different subtypes of rhinoviruses. These results indicated that 9A5 was a universal affinity antibody and that 2C01 contained a universal affinity epitope.

### Example 16: Use of monoclonal antibody 9A5 to detect human rhinovirus infection and titer

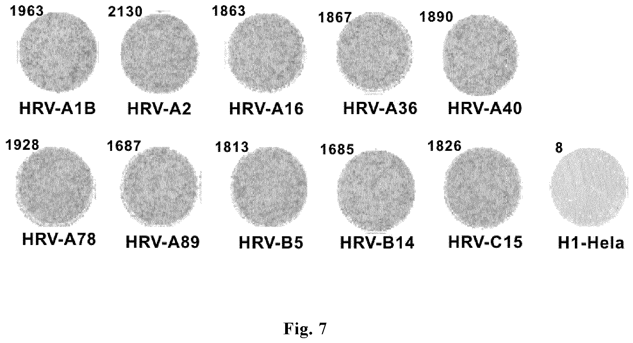

The monoclonal antibody 9A5 prepared in Example 14 was used to detect the titer of rhinovirus using a gradient dilution spot counting method. In this example, we selected 10 representative rhinoviruses in Example 4 to measure the infection titers. The specific steps were as in Example 5, and the experimental results were shown in Table 14 and Fig. 7.

**Table 14: Relationship between infection dose of rhinovirus and number of infected cells in corresponding wells**

| Amount of rhinovirus stock solution (10⁻³ mL) | 100 | 20 | 4 | 0.8 | 0.16 | 0.032 |
|---|---|---|---|---|---|---|
| Number of HRV-A1B infected cells | 1963 | 933 | 279 | 70 | 25 | 6 |
| Number of HRV-A2 infected cells | 2130 | 1092 | 344 | 121 | 35 | 9 |
| Number of HRV-A16 infected cells | 1863 | 902 | 317 | 62 | 27 | 7 |
| Number of HRV-A36 infected cells | 1867 | 899 | 213 | 54 | 18 | 6 |
| Number of HRV-A40 infected cells | 1890 | 901 | 245 | 66 | 21 | 8 |
| Number of HRV-A78 infected cells | 1928 | 931 | 301 | 76 | 26 | 7 |
| Number of HRV-A89 infected cells | 1687 | 763 | 115 | 34 | 12 | 5 |
| Number of HRV-B5 infected cells | 1813 | 884 | 291 | 51 | 21 | 7 |
| Number of HRV-B14 infected cells | 1685 | 791 | 100 | 29 | 10 | 5 |
| Number of HRV-C15 infected cells | 1826 | 832 | 273 | 66 | 22 | 6 |

According to the results in Table 14 and Fig. 7, it could be seen that the viral titers of 10 representative rhinoviruses (HRV-A1B, HRV-A2, HRV-A16, HRV-A36, HRV-A40, HRV-A78, HRV-A89, HRV-B5, HRV-B14 and HRV-C15) were 8.75×10⁴ (IU/mL), 1.513×10⁵ (IU/mL), 7.75×10⁴ (IU/mL), 6.75×10⁴ (IU/mL), 8.25×10⁴ (IU/mL), 9.5×10⁴ (IU/mL), 2.875×10⁴ (IU/mL), 6.375×10⁴ (IU/mL), 2.5×10⁴ (IU/mL) and 8.25×10⁴ (IU/mL), respectively. The results of this example showed that the monoclonal antibody 9A5 could be used to determine the infection titers of different representative rhinoviruses.

### Example 17: Use of monoclonal antibody 9A5 to detect the titer of neutralizing antibody against human rhinovirus

By using the enzyme-linked immunospot method, in this example, the monoclonal antibody 9A5 prepared in Example 14 was used to detect the titer of neutralizing antibody against human rhinovirus. The specific steps were as in Example 7.

In this example, 6 samples of polyantiserum from immunized mouse prepared in Example 5 were randomly selected and used for the detection of neutralizing antibody against human rhinovirus. The detection results were shown in Table 15. The results showed that the monoclonal antibody 9A5 could be used to detect the titer of neutralizing antibody against human rhinovirus; the polyantiserum from immunized mouse could neutralize HRV-A2, HRV-B14 or HRV-C15.

**Table 15: Detection results of the titer of neutralizing antibody in the polyantiserum from immunized mouse**

| Immunogen | Mouse No. | neutralizing antibody titer against HRV-A2 | neutralizing antibody titer against HRV-B14 | neutralizing antibody titer against HRV-C15 |
|---|---|---|---|---|
| HRV-A2 virus | Mouse 1 | 512 | 0 | 0 |
| HRV-A2 virus | Mouse 2 | 1024 | 0 | 0 |
| HRV-B14 virus | Mouse 1 | 0 | 1024 | 0 |
| HRV-B14 virus | Mouse 2 | 0 | 256 | 0 |
| HRV-C15 virus | Mouse 1 | 0 | 0 | 128 |
| HRV-C15 virus | Mouse 2 | 0 | 0 | 1024 |

### Example 18: Sequence determination of monoclonal antibody 9A5 light chain and heavy chain variable regions

10⁷ 9A5 mouse hybridoma cells were cultured in a semi-adherent culture. The adherent cells were blown up with a blowpipe for suspension, transferred to a new 4 mL centrifuge tube, and centrifuged at 1500 rpm for 3 minutes. The precipitated cells were collected, resuspended in 100 µL of sterile PBS buffer, transferred to a new 1.5 mL centrifuge tube, added with 800 µL of Trizol (purchased from Roche, Germany), mixed gently by inverting, and allowed to stand for 10 minutes. 200 µL of chloroform was added, shaken vigorously for 15 s, allowed to stand for 10 minutes, and centrifuged at 12000 rpm for 15 minutes at 4°C, the upper layer liquid was transferred to a new 1.5 mL centrifuge tube, added with an equal volume of isopropyl alcohol, mixed well, and allowed to stand for 10 minutes. After centrifugation was performed at 12,000 rpm for 5 minutes at 4°C, the supernatant was discarded, and the precipitate was vacuum dried at 60°C for 5 minutes. The transparent pellet was dissolved in 70 µL of DEPC H₂O, added with 1 µL of reverse transcription primer Oligo (dT) (12-18) (purchased from Promega) and 1 µL of dNTP, placed in a 72°C water bath for 10 minutes, and then immediately placed in an ice bath for 5 minutes, added with 20 µL of 5× reverse transcription buffer, 2 µL of AMV (10 U/µL, purchased from Promega), 1 µL of Rnasin (40 U/µL, purchased from Promega), mixed at 42°C to perform the reverse transcription of RNA into cDNA.

The light chain gene was isolated using the above cDNA as a template, MVkF-G1 (SEQ ID NO: 16) as an upstream primer, and MVkR-M13 (SEQ ID NO: 17) as a downstream primer. PCR amplification was performed to obtain a DNA fragment with a size of approximately 500 bp, the PCR conditions were: 95°C for 3 min, 23 cycles of (95°C for 15 s, 56°C for 30 s, 72°C for 30 s), and 72°C for 5 min. Sequencing was carried out after recovery. The sequence was determined to be the light chain sequence of 9A5 after alignment by blasting, in which the gene encoding the variable region was shown in SEQ ID NO: 18, and the amino acid sequence encoded thereby was shown in SEQ ID NO: 19.

The heavy chain gene was isolated using the above cDNA as a template, MVhF-C1 (SEQ ID NO:20) as an upstream primer, and MVhR-M13 (SEQ ID NO:21) as a downstream primer. PCR amplification was performed to obtain a DNA fragment with a size of approximately 500 bp, the PCR conditions were: 95°C for 3 min, 23 cycles of (95°C for 15 s, 56°C for 30 s, 72°C for 30 s), and 72°C for 5 min. Sequencing was performed after recovery. The sequence was determined to be the heavy chain sequence of 9A5 after alignment by blasting. The gene encoding the variable region was shown in SEQ ID NO: 22, and the amino acid sequence encoded thereby was shown in SEQ ID NO: 23.

In addition, the CDR sequences (SEQ ID NOs: 24 to 29) of the light chain and heavy chain of monoclonal antibody 9A5 were determined based on the IMGT database (http://www.imgt.org/IMGT_vquest/analysis).

Although certain specific embodiments of the present invention have been described in detail above, those skilled in the art will understand that, in light of all teachings that have been disclosed, various modifications and substitutions can be made in the details without departing from the spirit and gist of the present invention, and these changes are within the protection scope of the present invention. The scope of the present invention is limited only by the appended claims and any equivalents thereof.

## Claims

1. An isolated polypeptide or variant thereof, wherein the polypeptide consists of at least 13 consecutive amino acid residues of a human rhinovirus (HRV) 2C protein, and comprising amino acid residues at positions 151 to 163 of the protein, and, the variant differs from the polypeptide from which it is derived only by a substitution of one or several (e.g., 1, 2, 3, 4, 5, 6, or 7) amino acid residues, and retains the biological function of the polypeptide from which it is derived (e.g., inducing an antibody having specific binding activity to at least 2 (e.g., 2, 5, 10, 15, 20, 24) subtypes of HRV);
for example, the polypeptide consists of no more than 100 (e.g., no more than 95, no more than 90, no more than 85, no more than 80, no more than 75, no more than 70, no more than 65, no more than 60, no more than 55, no more than 50, no more than 45, no more than 40, no more than 35, or no more than 30) consecutive amino acid residues of the HRV 2C protein;
for example, the polypeptide consists of 13 to 30 (e.g., 13 to 25, 13 to 21, 13 to 17, 13 to 15) consecutive amino acid residues of the HRV 2C protein;
for example, the variant comprises a substitution (e.g., a conservative substitution) in an amino acid position corresponding to the position 156 and/or 159 of the HRV 2C protein;
for example, the polypeptide or variant thereof comprises a structure as set forth in YSLPPX₁PKX₂FDGY (SEQ ID NO: 52);
wherein, X₁ is selected from: (i) amino acid residues D, A, S, N, and (ii) amino acid residues that are conservatively substituted relative to (i);
X₂ is selected from: (i) amino acid residues Y, H, and (ii) amino acid residues that are conservatively substituted relative to (i);
for example, the HRV 2C protein has a sequence as set forth in SEQ ID NO: 9 or a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared thereto;
for example, the amino acid residues at positions 151 to 163 of the HRV 2C protein are as set forth in any one of SEQ ID NOs: 1, 30 to 34;
for example, the polypeptide comprises or consists of a sequence as set forth in any one of SEQ ID NOs: 1, 30 to 37.

2. A recombinant protein, which comprises the isolated polypeptide or variant thereof according to claim 1, and a carrier protein, wherein the recombinant protein is not a naturally occurring protein or fragment thereof;
for example, the polypeptide or variant thereof is optionally linked to a carrier protein via a linker (e.g., a rigid or flexible linker, such as a peptide linker comprising one or more glycines and/or one or more serines);
for example, the carrier protein is selected from the group consisting of CRM197 protein or fragment thereof, HBcAg or fragment thereof, WHcAg or fragment thereof, keyhole hemocyanin, human serum albumin, bovine serum albumin, bovine thyroglobulin, and ovalbumin.

3. The recombinant protein according to claim 2, wherein the carrier protein is HBcAg or fragment thereof, and the amino acids at positions 79 to 80 of HBcAg are replaced with the polypeptide or variant thereof;
optionally, the polypeptide or variant thereof is linked to HBcAg or fragment thereof via a linker;
for example, the fragment of HBcAg comprises or consists of aa 1-149 of HBcAg;
for example, the recombinant protein has an amino acid sequence as set forth in any one of SEQ ID NOs: 5, 44 to 51.

4. An isolated nucleic acid molecule, which comprises a nucleotide sequence encoding the polypeptide or variant thereof according to claim 1, or the recombinant protein according to claim 2 or 3.

5. A vector, which comprises the isolated nucleic acid molecule according to claim 4.

6. A host cell, which comprises the isolated nucleic acid molecule according to claim 4 or the vector according to claim 5.

7. A method for preparing the polypeptide or variant thereof according to claim 1 or the recombinant protein according to claim 2 or 3, which comprises culturing the host cell according to claim 6 under an appropriate condition, and recovering the polypeptide or variant thereof or the recombinant protein from a cell culture.

8. A virus-like particle (VLP), wherein the isolated polypeptide or variant thereof according to claim 1 is displayed on its surface;
for example, the virus-like particle comprises or consists of a fusion protein, the fusion protein comprises the polypeptide or variant thereof according to claim 1, and a carrier protein; optionally, the polypeptide or variant thereof and the carrier protein are linked via a linker;
for example, the carrier protein is HBcAg protein or fragment thereof (e.g., aa 1-149 of HBcAg); for example, the amino acids at positions 79 to 80 of HBcAg are replaced with the polypeptide or variant thereof;
for example, the fusion protein has an amino acid sequence as set forth in any one of SEQ ID NOs: 5, 44 to 51.

9. A composition, which comprises the polypeptide or variant thereof according to claim 1, the recombinant protein according to claim 2 or 3, the isolated nucleic acid molecule according to claim 4, the vector according to claim 5, the host cell according to claim 6 or the VLP according to claim 8;
for example, the composition is a pharmaceutical composition or an immunogenic composition (e.g., a vaccine);
for example, the composition further comprises a pharmaceutically acceptable carrier and/or excipient (e.g., an adjuvant);
for example, the composition comprises one or more kinds of the polypeptide or variant thereof, and these kinds of polypeptide or variant thereof may be independent or tandem, modified or unmodified, conjugated to other protein, or unconjugated to other protein;
for example, the composition comprises one or more kinds of the recombinant protein;
for example, the composition comprises one or more kinds of the VLP.

10. Use of the polypeptide or variant thereof according to claim 1, the recombinant protein according to claim 2 or 3, the isolated nucleic acid molecule according to claim 4, the vector according to claim 5, the host cell according to claim 6 or the VLP according to claim 8 in the manufacture of a preparation for inducing an immune response against HRV in a subject and/or for preventing and/or treating an HRV infection or a disease associated with HRV infection (e.g., respiratory tract infection) in a subject;
for example, the preparation is a vaccine.

11. A method for inducing an immune response against HRV in a subject and/or for preventing and/or treating an HRV infection or a disease associated with HRV infection (e.g., respiratory tract infection) in a subject, which comprises: administrating the subject in need thereof an effective amount of the polypeptide or variant thereof according to claim 1, the recombinant protein according to claim 2 or 3, the VLP according to claim 8, or the composition according to claim 9.

12. A monoclonal antibody or antigen-binding fragment thereof, wherein the monoclonal antibody is capable of specifically binding to the polypeptide or variant thereof according to claim 1, the recombinant protein according to claim 2 or 3, or the VLP according to claim 8;
for example, the monoclonal antibody is capable of specifically binding to the amino acid residues at positions 151 to 163 of HRV 2C protein;
for example, the monoclonal antibody or antigen-binding fragment thereof is selected from the group consisting of Fab, Fab', F(ab')₂, Fd, Fv, dAb, complementarity determining region fragment, single chain antibody (e.g., scFv), murine antibody, humanized antibody, fully human antibody, chimeric antibody (e.g., human-mouse chimeric antibody), or bispecific or multispecific antibody.

13. The antibody or antigen-binding fragment thereof according to claim 12, which comprises:
(a) a heavy chain variable region (VH) comprising the following 3 complementarity determining regions (CDRs):
(i) a VH CDR1, which consists of the following sequence: SEQ ID NO: 27, or a sequence having a substitution, deletion or addition of one or several amino acids (e.g., a substitution, deletion or addition of 1, 2 or 3 amino acids) as compared thereto,
(ii) a VH CDR2, which consists of the following sequence: SEQ ID NO: 28, or a sequence having a substitution, deletion or addition of one or several amino acids (e.g., a substitution, deletion or addition of 1, 2 or 3 amino acids) as compared thereto, and
(iii) a VH CDR3, which consists of the following sequence: SEQ ID NO: 29, or a sequence having a substitution, deletion or addition of one or several amino acids (e.g., a substitution, deletion or addition of 1, 2 or 3 amino acids) as compared thereto,
and/or,
(b) a light chain variable region (VL) comprising the following 3 complementarity determining regions (CDRs):
(iv) a VL CDR1, which consists of the following sequence: SEQ ID NO: 24, or a sequence having a substitution, deletion or addition of one or several amino acids (e.g., a substitution, deletion or addition of 1, 2 or 3 amino acids) as compared thereto,
(v) a VL CDR2, which consists of the following sequence: SEQ ID NO: 25, or a sequence having a substitution, deletion or addition of one or several amino acids (e.g., a substitution, deletion or addition of 1, 2 or 3 amino acids) as compared thereto, and
(vi) a VL CDR3, which consists of the following sequence: SEQ ID NO: 26, or a sequence having a substitution, deletion or addition of one or several amino acids (e.g., a substitution, deletion or addition of 1, 2 or 3 amino acids) as compared thereto;
preferably, the CDR described in any one of (i) to (vi) is defined according to the IMGT numbering system;
preferably, the substitution described in any one of (i) to (vi) is a conservative substitution.

14. The antibody or antigen-binding fragment thereof according to claim 12 or 13, which comprises:
(a) the following three heavy chain CDRs: a VH CDR1 having a sequence as set forth in SEQ ID NO: 27, a VH CDR2 having a sequence as set forth in SEQ ID NO: 28, and a VH CDR3 having a sequence as set forth in SEQ ID NO: 29; and/or, the following three light chain CDRs: a VL CDR1 having a sequence as set forth in SEQ ID NO: 24, a VL CDR2 having a sequence as set forth in SEQ ID NO: 25, and a VL CDR3 having a sequence as set forth in SEQ ID NO: 26;
or,
(b) three CDRs as comprised in the heavy chain variable region (VH) as set forth in SEQ ID NO: 23; and/or, three CDRs as comprised in the light chain variable region (VL) as set forth in SEQ ID NO: 19;
preferably, the three CDRs comprised in the VH and/or the three CDRs comprised in the VL are defined by the Kabat, IMGT or Chothia numbering system.

15. The antibody or antigen-binding fragment thereof according to any one of claims 12 to 14, which comprises:
(a) a heavy chain variable region (VH), which comprises an amino acid sequence selected from the following:
(i) a sequence as set forth in SEQ ID NO: 23;
(ii) a sequence having a substitution, deletion or addition of one or several amino acids (e.g., a substitution, deletion or addition of 1, 2, 3, 4 or 5 amino acids) as compared to the sequence set forth in SEQ ID NO: 23; or
(iii) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in SEQ ID NO: 23;
and
(b) a light chain variable region (VL), which comprises an amino acid sequence selected from the following:
(iv) a sequence as set forth in SEQ ID NO: 19;
(v) a sequence having a substitution, deletion or addition of one or several amino acids (e.g., a substitution, deletion or addition of 1, 2, 3, 4 or 5 amino acids) as compared to the sequence set forth in SEQ ID NO: 19; or
(vi) a sequence having a sequence identity of at least 80%, at least 85%, at least 90%, at least 91%, at least 92%, at least 93%, at least 94%, at least 95%, at least 96%, at least 97%, at least 98%, at least 99%, or 100% as compared to the sequence set forth in SEQ ID NO: 19;
preferably, the substitution described in (ii) or (v) is a conservative substitution;
preferably, the antibody or antigen-binding fragment thereof comprises: a VH comprising the sequence as set forth in SEQ ID NO: 23 and a VL comprising the sequence as set forth in SEQ ID NO: 19.

16. The antibody or antigen-binding fragment thereof according to any one of claims 12 to 14, which is humanized;
preferably, the antibody or antigen-binding fragment thereof comprises a framework sequence derived from a human immunoglobulin;
preferably, the antibody or antigen-binding fragment thereof comprises: a heavy chain framework sequence derived from a human heavy chain germline sequence, and a light chain framework sequence derived from a human light chain germline sequence.

17. The antibody or antigen-binding fragment thereof according to any one of claims 12 to 16, which further comprises a constant region derived from a murine or human immunoglobulin;
preferably, the heavy chain of the antibody or antigen-binding fragment thereof comprises a heavy chain constant region derived from a murine or human immunoglobulin (e.g., IgG1, IgG2, IgG3 or IgG4), and the light chain of the antibody or antigen-binding fragment thereof comprises a light chain constant region (e.g., a constant region of κ or λ chain) derived from a murine or human immunoglobulin.

18. A monoclonal antibody or antigen-binding fragment thereof, which is produced by hybridoma cell strain 9A5, or a cell strain derived from hybridoma cell strain 9A5, wherein the hybridoma cell strain 9A5 is deposited in the China Center for Type Culture Collection (CCTCC), and has the deposit number of CCTCC NO. C2021141.

19. The antibody or antigen-binding fragment thereof according to any one of claims 12 to 18, wherein the antibody or antigen-binding fragment thereof specifically recognizes at least 2 (e.g., 2, 5, 10, 15, 20, 24) subtypes of HRV;
for example, the antibody or antigen-binding fragment thereof specifically recognizes one or more selected from the group consisting of HRV-A1B, HRV-A2, HRV-A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV-C15, and HRV-C23.

20. An isolated nucleic acid molecule, which encodes the antibody or antigen-binding fragment thereof according to any one of claims 12 to 19, or heavy chain variable region and/or light chain variable region thereof.

21. A vector, which comprises the isolated nucleic acid molecule according to claim 20; preferably, the vector is a cloning vector or an expression vector.

22. A host cell, which comprises the isolated nucleic acid molecule according to claim 20 or the vector according to claim 21.

23. Hybridoma cell strain 9A5, which is deposited in the China Center for Type Culture Collection (CCTCC) and has the deposit number of CCTCC NO. C2021141.

24. A method for preparing the antibody or antigen-binding fragment thereof according to any one of claims 12 to 19, which comprises culturing the host cell according to claim 22 or the hybridoma cell according to claim 23 under a condition that allows expression of the antibody or antigen-binding fragment thereof, and recovering the antibody or antigen-binding fragment thereof from a cell culture of the host cell or hybridoma cell.

25. A conjugate, which comprises the antibody or antigen-binding fragment thereof according to any one of claims 12 to 19, and a detectable label connected to the antibody or antigen-binding fragment thereof;
for example, the detectable label is selected from the group consisting of enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide and biotin.

26. A kit, which comprises the antibody or antigen-binding fragment thereof according to any one of claims 12 to 19 or the conjugate according to claim 25;
for example, the kit comprises the conjugate according to claim 25;
for example, the kit comprises the antibody or antigen-binding fragment thereof according to any one of claims 12 to 19, and a second antibody that specifically recognizes the antibody or antigen-binding fragment thereof; optionally, the second antibody further comprises a detectable label such as enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide, or biotin.

27. A method for detecting the presence or level of HRV in a sample, which comprises using the antibody or antigen-binding fragment thereof according to any one of claims 12 to 19 or the conjugate according to claim 25;
for example, the method is an immunological detection, such as an immunoblot, an enzyme immunoassay (e.g., ELISA), a chemiluminescent immunoassay, a fluorescent immunoassay, or a radioimmunoassay;
for example, the HRV comprises at least 2 (e.g., 2, 5, 10, 15, 20, 24) subtypes; for example, the HRV comprises one or more selected from the group consisting of HRV-A1B, HRV-A2, HRV-A9, HRV-A16, HRV-A21, HRV-A23, HRV-A29, HRV-A34, HRV-A36, HRV-A40, HRV-A43, HRV-A49, HRV-A54, HRV-A59, HRV-A77, HRV-A78, HRV-A89, HRV-B5, HRV-B6, HRV-B14, HRV-B17, HRV-C11, HRV-C15, and HRV-C23;
for example, the method comprises using the conjugate according to claim 25;
for example, the method comprises using the antibody or antigen-binding fragment thereof according to any one of claims 12 to 19, and the method further comprises using a second antibody carrying a detectable label (e.g., enzyme (e.g., horseradish peroxidase or alkaline phosphatase), chemiluminescent reagent (e.g., acridinium ester, luminol and derivative thereof, or ruthenium derivative), fluorescent dye (e.g., fluorescein or fluorescent protein), radionuclide or biotin) to detect the antibody or antigen-binding fragment thereof.

28. Use of the antibody or antigen-binding fragment thereof according to any one of claims 12 to 19 in the manufacture of a detection reagent for detecting the presence or level of HRV in a sample, and/or for diagnosing whether a subject is infected with HRV;
preferably, the detection reagent detects the presence or level of HRV in the sample by the method according to claim 27;
preferably, the sample is a body fluid sample (e.g., respiratory secretion, whole blood, plasma, serum, salivary excretion or urine) from a subject (e.g., a mammal, preferably a human).

29. A method for detecting the neutralizing activity of an HRV-neutralizing antibody or screening an HRV-neutralizing antibody, which comprises using the antibody or antigen-binding fragment thereof according to any one of claims 12 to 19 or the conjugate according to claim 25;
for example, the method is an immunological detection method, such as an immunoblot, an enzyme immunoassay (e.g., ELISA), a chemiluminescent immunoassay, a fluorescent immunoassay, or a radioimmunoassay;
for example, the method comprises: (i) incubating a test sample containing an antibody to be tested with HRV; (ii) incubating the product of step (i) with a cell; (iii) incubating the antibody or antigen-binding fragment thereof or the conjugate with a cell product of step (ii) (e.g., the cell product of step (ii) treated with membrane permeabilization); (iv) determining the number of cells labeled by the antibody or antigen-binding fragment thereof or the conjugate, and thereby determining the neutralizing activity of the antibody to be tested against HRV.

30. A pharmaceutical composition, which comprises the antibody or antigen-binding fragment thereof according to any one of claims 12 to 19, and a pharmaceutically acceptable carrier and/or excipient.

31. Use of the antibody or antigen-binding fragment thereof according to any one of claims 12 to 19, the isolated nucleic acid molecule according to claim 20, the vector according to claim 21, the host cell according to claim 22 or the hybridoma cell according to claim 23 in the manufacture of a medicament for preventing and/or treating an HRV infection or a disease related to HRV infection (e.g., respiratory tract infection) in a subject;
preferably, the subject is a mammal, such as a human;
preferably, the antibody or antigen-binding fragment thereof is used alone or in combination with an additional pharmaceutically active agent.

32. A method for preventing and/or treating an HRV infection or a disease associated with HRV infection (e.g., respiratory tract infection) in a subject (e.g., a human), which comprises: administrating a subject in need thereof an effective amount of the antibody or antigen-binding fragment thereof according to any one of claims 12 to 19 or the pharmaceutical composition according to claim 30.
